# EUROPEAN PATENT APPLICATION

(11) **EP 3 929 281 A1**
(43) Date of publication of application: **29.12.2021**
(21) Application number: 20181946.3
(22) Date of filing: 24.06.2020
(51) Int. Cl.: C12N 5/079, A61K 35/30

(54) **CELL CONSTRUCT COMPRISING SCHWANN CELLS OR SCHWANN CELL-LIKE CELLS AND A BIOCOMPATIBLE MATRIX**

(71) Applicant: Fachhochschule Technikum Wien, 1060 Wien (AT)
(72) Inventor: TEUSCHL, Andreas, 1060 Wien (AT); HROMADA, Carina, 1060 Wien (AT)
(74) Representative: SONN Patentanwälte OG

(57) **Abstract**

The present invention discloses a method for producing a cell construct comprising Schwann cells or Schwann cell-like cells and a biocompatible matrix, wherein Schwann cells or Schwann cell-like cells or precursor cells of Schwann cells or Schwann cell-like cells are contacted with the biocompatible matrix and subjected to cultivation, wherein cultivation is at least partially performed by administering mechanical stimulation on the cells in contact with the biocompatible matrix.

## Description

The present invention relates to cell constructs comprising mechanically aligned Schwann cells or Schwann cell-like cells and a biocompatible matrix, methods for producing such cell constructs and uses of such cell constructs.

Schwann cells are the main glial cells of the peripheral nervous system, which wrap around axons and produce the myelin sheath necessary for saltatory signal conduction. Of note, one Schwann cell can only produce myelin for a single segment of an axon, referred to as an internode. The individual myelin segments are interrupted by a short unmyelinated segment called node of Ranvier. Furthermore, in order to resist mechanical stresses associated not only with developmental growth, but also with daily activities, peripheral nerves are protected by three layers of connective tissue: the endoneurium, perineurium and epineurium. The endoneurium is mainly composed of oriented collagen fibers and encloses the individual nerve fibers. The perineurium is the protective sheath that is wrapped around nerve fascicles, which is a bundle of several nerve fibers. These nerve fascicles are bundled in the connective tissue layer called epineurium, which is mostly composed of collagen, fibroblasts, vasculature and fat tissue, and contributes to the peripheral nerve's tensile strength.

Schwann cells also secrete a basal lamina that surrounds the axons, providing not only structural but also mechanical support. This basal lamina is a network mainly composed of collagen and laminin, which are crosslinked by proteoglycans and connect the extracellular space with the Schwann cell plasma membrane.

In contrast to the central nervous system, peripheral nerves have a remarkable regenerative capacity, yet, full intrinsic recovery is not sufficient to functionally recover severely damaged nerves, typically affecting larger nerve lesions > 4 cm. Thus, microsurgical interventions become necessary, including end-to-end suturing, autografts and allografts, as well as the use of nerve guidance conduits (NGCs). Direct end-to-end suturing of correctly aligned nerve stumps under tension-free conditions is the method of choice for smaller nerve lesions (< 5 mm). If tension-free suturing is not applicable, i.e. typically for larger nerve defects of up to 4 cm, the use of nerve autografts is considered the current gold standard. The main advantage of nerve autografts is their morphologically native structure, which provides not only guiding structures, but also Schwann cells producing extracellular matrix (ECM) molecules favourable for outgrowing axons towards the distal nerve stump. However, the use of autografts is limited to purely sensory nerves (mostly the sural nerve) in order to not induce loss of motor function at the donor site, but repair of motor nerve injuries using sensory nerve autografts cannot adequately regenerate the neurological deficit. If the nerve lesion requires a graft length exceeding available autografts, the use of allografts are clinical alternatives. In contrast to autografts, allograft tissue from human cadavers presents an unlimited source for nerve repair and is furthermore readily available in different lengths and diameters. Although this type of graft enhances nerve regeneration due to the native structural characteristics of neural tissue, it requires the use of immunosuppression to prevent rejection of the graft. To avoid the use of immunosuppression, commercially available decellularized nerve allografts have been developed, such as the Avance^{®} Nerve Graft from AxoGen. One major limitation of such acellular allografts is that they lack living SCs and therefore only provide the ECM of an uninjured, myelinated nerve, which differs strongly from that of a regenerating nerve and consequently does not sufficiently enhance axonal regeneration. One probable explanation is the time lapse between SC ingrowth from the distal and proximal nerve stump and the formation of bands of Büngner, which allow fast axonal regeneration and will be provided with the present invention. Especially in long defects, the long timespan between initial injury and contact of activated SCs with regenerating axons often results in senescence of SCs, impaired axonal regeneration and target innervation and therefore unsatisfying clinical results.

Direct nerve repair is the method of choice to approximate nerve defects shorter than 5 mm. Optimal recovery requires correct alignment of the nerve stumps as well as a tension-free approximation with a minimum number of stitches.

In cases where end-to-end suturing is not applicable, nerve autografts still remain the gold standard in treating peripheral nerve defects of up to 5 cm. The use of nerve autografts is limited to purely sensory nerves, such as the sural nerve, and associated with various disadvantages and poor functional recovery. These include donor site morbidity and mismatch, limited availability of suitable autografts, putative neuroma formation, and the necessity of an additional incision. Using sensory nerves for the repair of motor nerve injuries is often complicated by discrepancies in axon as well as Schwann cell characteristics of motor and sensory nerves, which potentially limits functional recovery of the injured nerve. For longer nerve gaps, allografts may present a suitable alternative.

Nerve allografts are applicable even for nerve injuries resulting in lesions of more than 5 cm in length, and yield functional recovery similar to that of nerve autografts. Reinnervation of the target organ via nerve allografts is achieved by donor Schwann cells that enable axonal regrowth from the proximal to the distal end. Although avoiding donor site morbidity, allogeneic nerve grafts require the temporary use of immunosuppression, which is associated with increased susceptibility to infections and tumour formation. Apart from that, the major advantage of an allogenic nerve graft over an autologous one is the ability to use the same nerve type that is to be repaired in the host, i.e. pure sensory, mixed sensory-motor, or pure motor nerves, thereby yielding improved recovery.

In order to overcome the limitations and detrimental side-effects of auto- and allografts, tissue engineering strategies for peripheral nerve repair have gained immense interest in the last decades. The main focus of neural tissue engineering is on providing guidance cues for regenerating axons, with special research interest being laid on the development and refinement of NGCs. NGCs provide a controlled microenvironment to guide regenerating nerve fibers and have several advantages over the use of autografts, such as lack of donor-site morbidity and limited neuroma formation. However, although some clinical success in peripheral nerve repair has been observed, the regenerative potential of these hollow NGCs is inferior to autografts and only yields poor functional recovery, while being furthermore limited to nerve gaps < 4 cm.

Since the self-regeneration capacity of peripheral nerves is limited in its ability to repair significant nerve gaps, the construction of satisfactory artificial transplants is of paramount importance. Tissue engineering strategies for peripheral nerve repair aim at establishing an alternative to nerve autografting especially for longer nerve gaps with superior functional recovery to overcome the limitations and detrimental side effects associated with autogenous nerve grafts. The success of tissue engineered constructs typically depends on three main components, also known as the tissue engineering triad: the type of biomaterial used as a scaffold, the choice of an appropriate cell type, as well as a suitable stimulation thereof, e.g. via growth factors. The main focus of neural tissue engineering is on providing guidance cues for regenerating axons, with special research interest being laid on the development of aforementioned nerve guidance conduits. Initially, natural or synthetic NGCs alone have been applied to provide guidance to regenerating axons by directing the axonal growth cone that is formed within the conduit towards the distal end. However, the use of these hollow tubes failed to regenerate larger nerve defects, thereby leading to the development and additional use of intraluminal NGC fillers. These fillers are designed to promote glial cell migration and for this purpose often include alignment of the biomaterial itself as well as supplementation of neurotrophic factors and ECM molecules to offer cell attachment sites.

EP 1 610 834 B1 discloses self-aligning tissue growth guides. WO 92/03536 A1 relates to the auto transplantation of Schwann cells to promote nervous system repair. US 2017/0135802 A1 describes 3D printed patient-specific conduits for treating complex peripheral nerve injury. US 2013/0184724 A1 reports a peripheral nerve growth scaffold. US 2005/0226856 A1 discloses autogenic living scaffolds. EP 3 235 470 A1 describes a biohybrid for regenerating neural tracts. EP 1 915 165 B1 reports an elastomeric polymer film substrate for influencing the morphology of cells. WO 2019/166087 A1 discloses implantable nerve guidance conduit for nerve repair. US 2016/0166733 A1 describes engineered neural tissue. US 2006/0233855 A1 discloses controlling 3D neurite outgrowth using PEG-fibrinogen hydrogels for regenerating nerves. US 2007/0239080 A1 discloses a method of enhancing the regeneration of injured nerves which comprises the step of administering an effective exposure of pressure pulses or acoustic shock waves in a pulse or wave pattern to the Zone of injury of the nerve during the regeneration process. US 2013/0110138 A1 relates to 3D scaffolds which may be filled with fibrin gels or Schwann cells for regeneration of nerves. US 2002/0042128 A1 discloses "electroprocessed fibrin" and Schwann cells embedded in such fibrin matrix. CN 106729980 A refers to a chitosan film conduit with a fibrin hydrogel and Schwann cells for manufacturing a bionic nerve graft. WO 2008/140413 A1 discloses the use of fibrin sealants for axon regeneration and observes the behaviour of Schwann cells in fibrin containing conduits. Biscola et al. (J. Ven. Anim. Tox. Trop. Dis. 23 (2017), 13; doi: 10.1186/s40409-017-0103-1) review the use of fibrin sealants for nervous system treatments following injury and disease. Schuh et al. (Tiss. Engin. A 24 (2018), 1332-1340) disclose an optimised collagen-fibrin blend engineered neural tissue, which promotes peripheral nerve repair.

It is an object of the present invention to provide cell constructs comprising Schwann cells or Schwann cell-like cells, which can be used for the treatment of nerve injuries or as appropriate cell/tissue models. Preferably, the present invention should provide cell constructs, which enable excellent nerve repair when applied either alone or in combination with a NGC as an intraluminal filler after injury also for longer distances, e.g. to overcome nerve gaps of more than 2 cm. It is a further object to provide constructs useable in nerve surgery which are as homogeneous as possible (especially when involving natural material) and reproducible so as to provide cell construct structures which can be marketed and applied in standard nerve surgery.

Therefore, the present invention provides a method for producing a cell construct comprising Schwann cells or Schwann cell-like cells and a biocompatible matrix, wherein Schwann cells or Schwann cell-like cells or precursor cells of Schwann cells or Schwann cell-like cells are contacted with the biocompatible matrix and subjected to cultivation, wherein cultivation is at least partially performed by administering mechanical stimulation on the cells in contact with the biocompatible matrix.

The present invention provides preferably cell constructs comprising primary Schwann cells, isolated from tissues, or Schwann cell-like cells differentiated from other cell types such as mesenchymal stem cells, fibroblasts or induced pluripotent stem cells etc., and a biocompatible matrix, wherein Schwann cells or Schwann cell-like cells or precursor cells of Schwann cells or Schwann cell-like cells are contacted with the biocompatible matrix and subjected to cultivation, wherein cultivation is at least partially performed by administering mechanical stimulation on the cells in contact with the biocompatible matrix.

The present invention provides a method to provide Schwann cell or Schwann cell-like cell containing constructs, which effectively assist in neural surgery, especially neural surgery after injury.

Peripheral nerve injury elicits the process of Wallerian degeneration and induces a Schwann cell repair response: the axon and myelin sheath degenerate, Schwann cells become activated to proliferate and dedifferentiate into repair Schwann cells, which is accompanied by the upregulation of pro-regenerative genes (BDNF, NCAM, p75, neuregulins, etc) and downregulation of myelin-associated genes (Krox20, Sox10, MBP, P0, etc). Macrophages are recruited for phagocytosis of axonal and myelin debris. The neuronal reaction includes chromatolysis, which degrades Nissl bodies, and cell swelling. Importantly, Schwann cells align into so-called bands of Büngner, which serve as a guidance bridge for regenerating axon sprouts. Therefore, Schwann cells proliferate and elongate about three-fold to form this regeneration tracks. (Gomez-Sanchez et al. 2017, The Journal of Neuroscience, 37 (37) : 9086-9099). Upon successful reinnervation, Schwann cells again differentiate into myelinating cells.

With the present invention, such artificially created bands of Büngner constructs are provided, which promote axon sprouts after nerve injury and in the course of neural surgery.

In the present invention, artificial bands of Büngner are provided wherein Schwann cells or Schwann cell-like cells in a biocompatible scaffold are trained mechanically by mechanical tension. The Schwann cells in the scaffold construct thereby become oriented and connected to establish phenotypical bands of Büngner and show a change in gene expression (as proven by gene expression analysis). The cells used for the preparation of constructs of the present invention are Schwann cells or Schwann cell-like cells and precursors of such cells which can mature into Schwann cells or Schwann cell-like cells. Such cells are well available to persons skilled in the art.

As used herein, the term "Schwann cell" refers to a cell that express one or more Schwann cell marker, which include LRRTM4, CDH1, FABP7, BDNF, UNCB5, SOSTDC1, OLIG1, PLAT, KCNJ10, SHH, NTN1, GDNF, ERBB3, GAP43, SOX10, S100, GFAP, POU3F1, PMP22, MBP, AQP4, MPZ, NGFR, NFATC4, MOG, IFNG, MAL, NTF3, TGFB1, CD9, CD81, CD44, CD98, CD49E, CD49D, TYRP1, ENTHD1, NTSE, HTR2B, NOV, IL8, SLC16A6, CDKN2A, PLP2, S100A6, AQP9, and CDH19, preferably TYRP1, CD44, ENTHD1, NTSE, HTR2B, NOV, IL8, SLC16A6, and CDKN2A. The Schwann cell can be a myelinating Schwann cell or a non-myelinating Schwann cell. In certain embodiments, the Schwann cells are capable of maintaining and regenerating axons of the neurons in the peripheral nervous system (e.g., maintenance of healthy axons). In preferred embodiments, the Schwann cells are capable of forming the myelin sheath. In certain embodiments, the Schwann cells are capable of forming Remak bundles.

A "Schwann cell precursor" refers to a cell that express one or more Schwann cell precursor marker, which includes SOX10, GAP43, BLBP, MPZ, Dhh, P75NTR, CD49D, TFAP2, CDH19, CD44, ERBB3, POU3F1, GFAP, CALCB, GRP116, TSPYL5, ITPKA, SLC17A6, SYPL2, LOC100128252, ANGPTL7, LOC728978, ZNF502, SLC16A6, LPL, SLC30A2, and SLC10A4, preferably CALCB, GRP116, TSPYL5, ITPKA, SLC17A6, SYPL2, LOC100128252, ANGPTL7, LOC728978, and ZNF502. Under suitable maturation conditions, Schwann cell precursors can become Schwann cells.

Traditionally, Schwann cells are primary Schwann cells isolated as fully differentiated cells from tissue. Schwann cells can also be obtained from iPS cells (e.g. Kim et al., J. Stem cell Rep. 8 (2017), 1717-1726) or from other sources, such as adipose tissue. For example, functional Schwann cell-like cells can also be obtained from mesenchymal stem cells (MSCs), including adipose-derived stem cells (e.g. Faroni et al., Neural Regen. Res. 9 (2014), 1341-1346, Xie et al., Cell Cycle 16 (2017), 841-851, Himeno et al., BioMedRes. Int (2013), 259187).

Methods for providing Schwann cells, Schwann (cell)-like cells or precursors thereof are well available in the art (recently summarised e.g. in US 2019/0331666 A1, US 2019/0264173 A1, Sullivan et al., Int. J. Mol. Sci. 17 (2016) 2101; doi:10.3390/ijms17122101). For example, Schwann cells can be obtained from in vitro differentiation of stem cells (e.g., human stem cells). In preferred embodiments, the stem cell is a human stem cell. Examples of human stem cells include human embryonic stem cells (hESC), human pluripotent stem cell (hPSC), human induced pluripotent stem cells (hiPSC), human parthenogenetic stem cells, primordial germ cell-like pluripotent stem cells, epiblast stem cells, F-class pluripotent stem cells, somatic stem cells, cancer stem cells, or any other cell capable of lineage specific differentiation. In certain preferred embodiments, the human stem cell is a human pluripotent stem cell. In certain embodiments, the human stem cell is a human embryonic stem cell (hESC). In further preferred embodiments, the human stem cell is a human induced pluripotent stem cell (hiPSC), however, the stem cells may also be non-human stem cells, including, but not limited to, mammalian stem cells, such as primate stem cells or stem cells from a rodent, a mouse, a rat, a dog, a cat, a horse, a pig, a cow, a sheep, etc.

Inducing differentiation of stem cells (e.g., hPSC) to one type of neural lineage can be performed by use of (dual) SMAD inhibition. Furthermore, stem cells can be differentiated into neural crest lineage cells (e.g., nociceptors) by sequential inhibition of SMAD signalling followed by activation of Wnt signalling.

In preferred embodiments, the differentiation of stem cells to Schwann cells include three phases: in vitro differentiation of stem cells to cells expressing one or more neural crest lineage marker (neural crest lineage cells), in vitro differentiation of neural crest lineage cells to Schwann cell precursors, and in vitro differentiation or maturation of Schwann cell precursors to Schwann cells. Any suitable methods for in vitro differentiation of stem cells to neural crest lineage cells can be used in the first phase. In preferred embodiments, a population of stem cells is in vitro differentiated to a population of neural crest lineage cells, which is in vitro differentiated to a population of Schwann cell precursors, which is further induced in vitro to a population of Schwann cells.

Neural crest lineage marker include SOX10, p'75, HNK1, CD49D, ERBB3, TFAP2, SNAIL and SLUG.

In preferred embodiments, the neural crest lineage cells are in vitro differentiated from stem cells by inhibition of SMAD signalling and activation of Wnt signalling. In preferred embodiments, the method comprises contacting a population of stem cells (e.g., human stem cells) with one or more inhibitor of transforming growth factor beta (TGFβ)/Activin-Nodal signalling and one or more Wnt activator.

In preferred embodiments, the Schwann cell precursors are in vitro differentiated from neural crest lineage cells by inducing Schwann cell differentiation. In preferred embodiments, the method comprises contacting a population of neural crest lineage cells (e.g., the neural crest lineage cells derived from stem cells by inhibition of SMAD signalling and activation of Wnt signalling) with one or more Wnt activator and one or more FGF activator. In certain embodiments, the method comprises contacting a population of neural crest lineage cells (e.g., the neural crest lineage cells derived from stem cells by inhibition of SMAD signalling and activation of Wnt signalling) with one or more Schwann cell differentiation inducer.

In preferred embodiments, the Schwann cells are in vitro differentiated from Schwann cell precursors by enhancing Schwann cell differentiation. In certain embodiments, the method comprises contacting a population of Schwann cell precursors (e.g., the Schwann cell precursors cells derived from neural crest lineage cells by inducing Schwann cell differentiation) with one or more FGF activator, one or more Schwann cell differentiation inducer, and/or one or more Schwann cell differentiation enhancer. In certain embodiments, the method comprises contacting a population of Schwann cell precursors (e.g., the Schwann cell precursors cells derived from neural crest lineage cells by inducing Schwann cell differentiation) with one or more Schwann cell differentiation enhancer.

Examples for Schwann cells, Schwann cell-like cells, methods for their production and their performance in NGCs are disclosed in WO 2018/090002 A1 (e.g. by induction from stem cells), WO 2017/147491 A1 (e.g. from human fascicles), EP 3 196 305 A1 (e.g. by introducing into a somatic cell of a mammal at least SOX10 or KROX20 gene, or an expression product thereof), WO 2016/187135A1 (e.g. starting from epidermal neural crest stem cells), Kappos et al. (Stem Cells Dev. 24, (2015), 2127-2141), Cartarozzi et al. (Brain Res. Bull. 112 (2015), 14-24) Carriel et al. (J. Neural Eng. 10 (2013), 026022), Galla et al., Int. J. Artif. Organs 27 (2004), 127-136).

Kappos et al. compared the regenerative capacities of all of the aforementioned cell types in fibrin NGCs and showed that Schwann cell-like cells achieved better functional recovery than Schwann cells 12 weeks after transplantation to bridge a 1 cm rat sciatic nerve gap. However, although Schwann cell-like cells yielded better motor function recovery, the group also showed inferior remyelination as well as less myelinated axons in the medial and distal stumps compared to Schwann cells. Similarly, Cartarozzi et al. elucidated sciatic nerve regeneration in rats using a synthetic NGC either empty, filled with fibrin, or filled with fibrin and mesenchymal stem cells. Motor function recovery was better in nerves treated with NGCs containing fibrin and mesenchymal stem cells, while myelin thickness and the total number of myelinated nerve fibres was not superior to the other groups. Likewise, Carriel et al. compared collagen NGCs filled with saline solution, with fibrin-agarose hydrogel, or with fibrin-agarose hydrogel and ASCs in a rat sciatic nerve defect of 1 cm. After 12 weeks, the poorest regenerative response was observed in rats treated with the saline-filled NGC, while the most promising results were achieved with the ASC-embedded conduit in terms of Schwann cell migration, remyelination, aligned axon regrowth to the distal site as well as sensory and motor function recovery. Galla et al. showed that synthetic conduits filled with Schwann cell embedded fibrin matrices enhanced rat facial nerve regeneration when compared to empty or fibrin matrix only NGCs. Furthermore, enrichment of the fibrin/Schwann cell matrix with leukemia inhibitory factor prevented hyperneurotisation.

Further embodiments of Schwann cells, Schwann cell-like cells and precursors thereof as well as their provision can be derived e.g. from the literature cited above, including US 2019/0331666 A1 and US 2019/0264173 A1.

Accordingly, the present invention shows that it is possible to condition regenerative Schwann cells by mechanical stimulation. The artificial bands of Büngner provided with the present invention are suitable to improve regeneration of peripheral nerve injuries and to improve in vitro assessment of bands of Büngner (e.g. as a research tool).

According to the present invention, the Schwann cell or Schwann cell-like cell containing constructs of a biocompatible matrix are subjected to mechanical stimulation.

As already stated above, the Schwann cells according to the present invention can be any form of Schwann cells suggested for use as Schwann cells or Schwann cell-like cells in the present field. According to a preferred embodiment, the cells used in the present cell constructs are obtained from the patient to whom the constructs are then to be delivered by surgery, i.e. autologous cells. Accordingly, the Schwann cells or Schwann cell-like cells or the precursor cells of Schwann cells or Schwann cell-like cells are preferably autologous cells obtained from the patient to which the construct is planned to be delivered.

The term "mechanotransduction" refers to the conversion of mechanical stimuli into a wide range of biochemical responses, mediated by a wide range of extra- and intercellular structures as well as molecules. The importance of mechanosensitivity has long been shown for the majority of cell types in the human body, and likewise, also Schwann cell mechanosensitivity is known to influence their migration, shape and myelination and in further consequence also development, maintenance and regeneration of the PNS (Rosso et al., Front. Mol. Neurosci. (2017), 10:345, pages 3-4) .

Projects have been suggested to gain insights into these molecular mechanisms and translate them into a regenerative strategy for spinal cord injury (see: a "Wings of Life" project (https://www.wingsforlife.com/de/forschung/mechanotransduktion-in-nervenzellen-einezukuenftige-strategie-fuer-die-regeneration-nach-rueckenmarksverletzung-2917/) for a potential application for targeted and improved axon regeneration in the future in spinal cord neurosurgery as a "dream of reinnervation after injury or disease"). However, even for spinal surgery, the use of mechanical forces is still in a very early stage, also due to the lack of appropriate model and the lack of understanding the mechanisms of this process.

The peripheral nervous system has a remarkable biomechanical resilience to sustain the variety of physiological mechanical stresses it is exposed to during regular daily activities, which can be attributed to the connective tissue (i.e. epineurium, perineurium and endoneurium) as well as the basal lamina surrounding single nerve fibers (Rosso et al., Front. Mol. Neurosci. (2017), 10:345, pages 1-2). Compositional changes and structural disarrangement of this basal lamina are associated with initiation and progression of nerve diseases due to the loss of their mechanoprotective effect. It is well known that SCs are affected in their morphogenesis by the stiffness of the surrounding matrix; however, how SCs sense mechanical forces such as strain, compression or shear and how they are affected thereby is not fully understood or simply unknown. Studies in literature manipulating pure SCs focus on variations of culture substratum or establishing disease models. In this regard, the group of Prof. Gupta worked on mimicking chronic nerve compression (CNC) injuries by application of hydrostatic compression or shear stress on SCs (Gupta et al. J Orthop Res 2005;23(5):1232-9; Frieboes et al. J Neurotrauma 2012;26(12) :2245-56) . They could show that results from in vivo CNC models regarding myelin degradation could be simulated with in vitro disease models (Lin et al. Ann Neurol 2012;72(1):112-23). Since SCs are not exposed to mechanical loading in healthy nerve tissue due to shielding by the ECM and morphological changes of the SC phenotype have not been reported in response to mechanical stimulation, it was not to expected that cells start to align and form bands of Büngner-like structures upon mechanical deformation as shown in this patent.

In fact (and surprisingly), the exact mechanisms of mechanotransduction in Schwann cell biology are largely unknown and, since little or nothing is known about the molecular signalling pathways activated by the mechanical forces, no practical applications have been presented so far in the field of neurosurgery in connection with Schwann cells (Belin et al., Front. Cell. Neurosci. (2017), 11:177).

As mentioned before, Schwann cells produce ECM composed of collagen and laminin, the basal lamina. It is believed that mechanical signals are transmitted through the ECM and the Schwann cells' basal side. Particularly, cell adhesion molecules (CAMs) that are formed between axons and Schwann cells as well as between the basal lamina and Schwann cells are considered as essential mechanosensors necessary for the transduction of mechanical signals received. CAMs between the ECM and the Schwann cell include G-protein coupled receptors, integrins and dystroglycan, while CAMs between axons and Schwann cells comprise myelin-associated glycoprotein (MAG), neural cell adhesion molecule (NCAM), N-Cadherin, integrin β1 and neurofascins. Among these, integrins play a vital role in mechanotransduction since they link ECM proteins to the actin cytoskeleton, mainly via so-called focal adhesions. Hence, forces on the ECM are transmitted via these focal adhesions, resulting in a cytoskeletal rearrangement of actin filaments, microtubules and intermediate filaments (Belin et al., Front. Cell. Neurosci. (2017), 11:177, pages 4-6; Ingber, FASEB J. 20, (2006), 811-827; Schwartz, Cold Spring Harb. Perspect. Biol. 2 (2010), pp. a005066-a005066).

With the present invention, it was shown that administering mechanical stimulation on Schwann cell constructs can enable the provision of advantageous cell constructs with high regenerative capacity and potential in neural surgery.

A mechanical deformation step refers to the event of active construct manipulation e.g. through application of external forces, gravity, electromagnetic forces, etc. that lead to alterations of the construct in configuration and/or structure. According to the present invention, a single ("one") mechanical deformation step therefore refers to the induction of a mechanical stimulus by substantial mechanical deformation of the construct ("substantial" meaning that the deforming is sufficient to elicit gene expression level changes in response to mechanical stimulation (e.g. downregulation of myelin gene MBP (Example 5, below), preferably also connected/combined with other (known) genetic markers for mechanical stimulation, e.g. upregulation of Sox10 and pro-regenerative markers).

Preferred devices for mechanical stimulation of the cell constructs according to the present invention are devices, wherein mechanical stimulation is performed as active deformation of the construct, such as the mentioned bioreactor system using magnetic force transmission. In this case, one mechanical deformation step refers to one stretch of the construct due to the applied mechanical loading using the described bioreactor system.

An illustrative example of such bioreactor wherein cells in a matrix are actively stimulated by mechanically deforming the matrix is disclosed in Heher et al. (Acta Biomater. 24 (2015), 251-265). Further examples for such cell culture bioreactors are disclosed in US 2004/005297 A1 and US 4,851,354 A using hydrostatic pressure, US 6,306,169 B1 using compressive strain in agarose gels, US 6,117,674 B1, US 5,858,783 A, US 5,846,807 A, using stretching of cells (and microgravity or simulated microgravity).

Biomaterials in tissue engineering act as temporary framework structures that support the generation of three-dimensional cellular tissue constructs. Therefore, biomaterials should be biocompatible, biodegradable and mimic the native ECM so as to provide a microenvironment that supports cell adhesion, proliferation, migration and differentiation. Scaffolds for peripheral nerve tissue engineering should furthermore be pliable, neuroconductive and neuroinductive, as well as porous to allow for vascularization. Basically, biomaterials can be divided into natural and synthetic ones. Prominent synthetic biomaterials include polyglycolic acid (PGA), poly (D,L-lactic-co-glycolic) acid (PLGA) and poly L-lactic acid (PLLA). Their main advantageous characteristics include mechanical strength, high degree of tuneability of scaffold properties and excellent reproducibility. However, synthetic biomaterials have increased risks of rejection due to reduced bioactivity as they may not adequately mimic the native ECM. Natural biomaterials include (ECM-derived) proteins, such as collagen, gelatin, fibrin, keratin, fibronectin, laminin, or silk fibroin; and polysaccharides, such as alginate, hyaluronic acid and chitosan. In contrast to synthetic scaffolds, natural ones resemble the native cellular environment, are bioactive and support cell adhesion and proliferation. Furthermore, they present favourable degradation characteristics allowing implanted and invading cells to replace the degrading scaffold with newly produced ECM. Nevertheless, they may have inferior mechanical properties and generation of natural scaffolds is prone to result in heterogeneous, poorly reproducible structures.

The use of fibrin hydrogels as a biomaterial in the field of tissue engineering has become increasingly popular in recent years. One major advantage of fibrin hydrogels is that they mimic native ECM structure and composition as it is a naturally occurring product in coagulation. Moreover, it is biocompatible and biodegradable, thereby meeting two of the most important requirements for tissue engineering scaffolds. Furthermore, fibrin promotes cell adhesion due to the presence of cell attachment sites as well as its ability to bind ECM molecules such as vitronectin and fibronectin. Specifically, these cell attachment sites consist of Arg-Gly-Asp (RGD) sequences, which serve as recognition sequences for integrin receptors on cell membranes, including α_{Iib}β₃, αᵥβ₁ and αᵥβ₃. In particular, Schwann cells are known to express the integrin receptor αᵥβ₈ that specifically binds to the fibrin RGD sequence. Hence, fibrin also presents a favourable environment for cell migration, proliferation and differentiation. Another important feature of fibrin for tissue engineering applications is the ability to mimic the properties of native tissues by adjusting stiffness as well as pore size through fine-tuning of fibrinogen and thrombin concentration. Apart from that, fibrin is also known to be proangiogenic, thereby facilitating vascularization of the implanted tissue engineered construct.

Fibrin is of particular interest for peripheral nerve tissue engineering as it has been shown to suppress myelin protein synthesis in Schwann cells, but to induce their proliferative repair phenotype. It was shown that the induction of the proliferative Schwann cell phenotype in fibrin was caused by increased phosphorylation of ERK1/2. Although an inhibitory effect of fibrin on nerve remyelination was reported, improved functional recovery when using fibrin constructs was also shown. Sciatic nerve regeneration was improved by fibrin-made NGCs compared to synthetic NGCs in terms of Schwann cell migration and thus also axonal regeneration distance. Synthetic NGCs filled with fibrin gels significantly enhanced regeneration of myelinated nerve fibres in a 1 cm sciatic nerve defect as compared with empty NGCs and furthermore yielded comparable results as nerve isografts. It was further investigated whether Schwann cell-embedded fibrin gels incorporated into synthetic NGCs further improve peripheral nerve regeneration in a 1 cm rat facial nerve lesion. Although recovery using autografts was superior, NGCs filled with Schwann cell/fibrin hydrogels significantly improved nerve regeneration compared to empty NGCs and NGCs seeded with Schwann cells in absence of fibrin. More recently, a fibrin-collagen blend filler with incorporated immortalized Schwann cells was used inside a silicone tube to bridge an 8 mm sciatic nerve defect in a rat model. Four weeks post implantation, axonal regeneration in the middle and distal part was significantly higher when using collagen-fibrin blend fillers compared to collagen fillers alone. When comparing fibrin NGCs applied either empty, seeded with Schwann cells, seeded with Schwann cell-like cells or filled with an autograft to bridge a 1 cm sciatic nerve lesion in rats, it was observed that after 16 weeks, conduits seeded with Schwann cell-like cells showed the most promising results, comparative to those of the autograft group.

The choice of appropriate cell types to be included into NGCs in peripheral nerve regeneration is in principle an intensively studied field. Ideally, transplanted cells should be easy to isolate with minimal donor site morbidity and show a high proliferation rate while not eliciting an immune response.

In the present invention, the choice of Schwann cells or Schwann cell-like cells has proven to be suitable to solve the objects according to the present invention. Although Schwann cells present several favourable features, such as the secretion of neurotrophic factors and basal lamina as well as assistance for resident Schwann cells in remyelination of regenerating nerves, they are also associated with some drawbacks. On the one hand, isolation and expansion of primary Schwann cells is tedious, and on the other hand involves sacrificing of a healthy (sensory) nerve associated with donor-site morbidity. Therefore, in some preferred embodiments, Schwann cells isolated from adipose tissue, Schwann cell-like cells (e.g. differentiated from mesenchymal stem cells, fibroblasts or induced pluripotent stem cells, etc.) or undifferentiated mesenchymal stem cells may be used according to the present invention instead and/or in combination with Schwann cells. Therefore, mesenchymal stem cells represent an abundant cell source that can be easily isolated from adipose tissue or bone marrow. These cells can not only be expanded rapidly, they are also immunoprivileged and known to be pro-angiogenic. Furthermore, these cells can be differentiated into Schwann cell-like cells, and in contrast to their undifferentiated state, might provide enhanced regenerative potential similar to primary Schwann cells without exhibiting their aforementioned drawbacks. In the following paragraphs, selected studies using these cell types are summarized shortly.

According to a preferred embodiment of the present method, the cultivation and mechanical stimulation is performed (at least partially) in a bioreactor.

A preferred embodiment of the present method comprises the following steps:
- providing a cell construct of a biocompatible matrix with Schwann cells or Schwann cell-like cells or precursor cells of Schwann cells or Schwann cell-like cells in a bioreactor,
- cultivation of the cell construct in the bioreactor
- exposing the cell construct to a mechanical stimulation in the bioreactor during cultivation by fixing the cell construct to a mechanical apparatus which applies strain onto the construct.

According to a preferred method, 250,000 to 15,000,000 cells, preferably from 500,000 to 10,000,000 cells, especially from 1,000,000 to 5,000,000 cells are contacted with the biocompatible matrix and subjected to cultivation.

According to a preferred embodiment, the cultivation in the method according to the present invention comprises differentiation of the precursor cells into Schwann cells or Schwann cell-like cells, wherein cultivation can at least partially be performed in the bioreactor.

Preferably, the biocompatible matrix is present in a three-dimensional form, preferably with a support structure resembling the three-dimensional form, especially wherein the three-dimensional form is a three-dimensional cylinder form, a three-dimensional cuboid form, or a three-dimensional ring form. In a specifically preferred embodiment, the biocompatible matrix or the cell construct is provided as a three-dimensional construct in an oblongness shape (e.g. in the form of a cylinder or a cuboid) with a length (being the longest extension of the three-dimensional construct) and a diameter (as the longest extension of an axis which lies normal to the length of the construct) with a ratio of length to diameter of from 2:1 to 100:1, preferably from 4:1 to 40:1, especially from 5:1 to 20:1.

In a preferred embodiment, the cultivation is performed for a duration of 3 to 60 days, preferably for a duration of 7 to 42 days, especially for a duration of 14 to 28 days. Preferred cultivation parameters, such as temperature or medium are those known for these types of cells. For example, typical cultivation parameters may be cultivation in a humidified atmosphere at 37°C, 5% CO₂ in a Dulbecco's Modified Eagle's Medium (DMEM), optionally comprising further components, such as fetal calf serum or antibiotics. Preferred culture media are disclosed in the example section below, e.g. the Schwann cell culture medium.

Preferably, the mechanical stimulation on the cells in contact with the biocompatible matrix during cultivation is performed at least for the time of a single mechanical deformation (step) to 24 h (preferably from 30 min to 12 h, especially from 1 to 6 h) at least once a day for a duration of 1 to 60 days, preferably for a duration of 3 to 42 days, especially for a duration of 5 to 42 days.

According to a preferred embodiment, the mechanical stimulation on the cells is performed by a static strain treatment or a ramp strain treatment, preferably by a ramp strain treatment. Static strain treatment is defined as a constant stretch/elongation of the construct by a defined length, whereas in ramp strain, the extent of stretch exerted on the construct is continuously increased. According to a specifically preferred embodiment, the mechanical stimulation is performed as a strain treatment of 1 to 50 %, preferably of 2 to 30 %, especially of 3 to 20 %, or as a treatment corresponding to a strain treatment of 1 to 50 %, etc. (i.e. applying the corresponding mechanical stimulus of a strain treatment (as e.g. measured by expressed markers)). For example, with the MagneTissue apparatus disclosed in Heher et al., 2015, preferred strain treatments are in the range of 2 %, 3 %, 10 %, 12, or 20 % (see also: example section, below).

Preferably, the method according to the present invention applies a step of electrical stimulation wherein the cell construct is at least partially exposed to electrical stimulation (see e.g. Koppes et al., Tissue Eng. Part A (2014), 20(3-4): 494-506). Preferred electrical stimulation is therefore performed by applying 10 to 200 mV/mm, preferably 25 to 150 mV/mm, especially 50 to 125 mV/mm, for 1 to 24 h, preferably from 2 to 12 h, especially from 3 to 8 h (as measured and performed in Koppes et al.).

It is specifically preferred to perform cultivation and mechanical stimulation until bands of Büngner structures have formed, leading to alignment of cells along the axis of strain. The term "Büngner bands" or "bands of Büngner" originally referred to the native structures observed in the living being. According to the present invention, the cell constructs are, of course, not completely identical to the physiological "bands of Büngner" but structures (closely) resembling the physiological bands of Büngner, i.e. being functionally equivalent to physiological bands of Büngner ("Büngner-like structures") at least in providing a growth guiding structure for axons or in enabling to overcome nerve gaps of more than 2 cm in human surgery. The present constructs may be provided as bands of Büngner or Büngner-like structures with longitudinally aligned/linear bands of interdigitating Schwann cells (or Schwann-like cells) that guide regrowing axons. Physiological bands of Büngner are further disclosed e.g. under https://neuromuscular.wustl.edu/pathol/bungner.htm; as of 28 December 2018).

The products obtained by the method according to the present invention are specifically suitable to provide cell constructs, which can be applied in human surgery, especially for nerve repair. Accordingly, suitable steps for finishing the cell constructs to be applicable in human surgery are applied, such as providing the constructs in combination with a pharmaceutically acceptable carrier (usually a transport or culture medium suitable to be applied also in human surgery, e.g. serum free culture/transport media) and wrapped in a transportable and/or storage-stable form. Preferred pharmaceutically acceptable carriers are Schwann cell media which are suitable for transplant fluids in human surgery (e.g. serum free media). The cell construct is preferably cultivated in a serum free medium or transferred into a serum free medium after cultivation and stimulation, especially 0.7% saline solution or sterile phosphate buffered saline (PBS; NaCl 137 mM, KCl 2.7 mM, Na₂HPO₄ 10 mM, KH₂PO₄ 1.8 mM). Preferably, the constructs according to the present invention are transferred to the surgical desk essentially direct from the bioreactor (with, if at all, only washing steps or exchange of medium for the cell constructs in order to apply the constructs to a human patient). The present method may therefore also comprise washing and/or formulation steps for adapting the cell construct to surgical use, preferably by removing the cell construct from a bioreactor after cultivation and stimulation and placing the cell construct in a pharmaceutically acceptable medium. If direct transfer in fresh form to the surgical desk is not possible, the constructs are provided in a storage-stable form, e.g. with a storage buffer for cell constructs, or in deep-frozen (or, in certain embodiments, even lyophilised) form.

In any way (i.e. direct (as fresh products or in storage-stable form), the cell constructs according to the present invention are prepared as prescribed by pharmaceutical good manufacturing practice (GMP). Accordingly, all manufacturing steps are performed in good pharmaceutical manufacturing process manner. For example, all steps necessary for safeguarding surgical applicability are applied, for example all steps are performed under sterile conditions. Accordingly, in the method according to the present invention preferably the biocompatible matrix with the cultivated cells, which have been stimulated mechanically, are subjected to formulation steps for adapting the cell construct to surgical use, preferably by working under aseptic conditions.

As already stated above, the present invention also relates to cell construct obtainable by a method according to the present invention. The present invention also relates to a cell construct comprising Schwann cells or Schwann cell-like cells and a biocompatible matrix, wherein the cell construct has a bands of Büngner-like structure.

In a preferred embodiment, the cell construct according to the present invention is a nerve guidance graft, especially alone or in combination with a nerve guidance conduit, preferably comprising a pharmaceutically acceptable carrier or being manufactured for neurosurgical use.

The cell constructs according to the present invention are specifically suited as medical/surgical devices and can be used in therapeutic treatment. The preferred medical use is, of course, neuro-surgery, especially in the treatment of nerve injuries.

Initially, peripheral nerve injuries have been classified into three main types: neurapraxia, axonotmesis, and neurotmesis. Neurapraxia is the mildest form of nerve damage characterized by loss of signal transduction due to degradation of the myelin sheath but otherwise intact axons and connective tissue. Axonotmesis is defined by axon and myelin damage, while the continuity of the surrounding connective tissue is largely preserved. The most severe type of nerve injury is neurotmesis, which is characterized by complete transection of the axons and connective tissues with poor chances of recovery.

Later, an extended classification was proposed into five degrees of injury with increasing severity (Table 1). Degree I and II correspond to neurapraxia and axonotmesis, respectively. In degree III-IV nerve injuries, the axon is damaged with increasing severity of connective tissue injury, ranging from damage solely of the endoneurium (degree III) to damaged endoneurium and perineurium (degree IV) and finally damage of all connective tissue layers (i.e. endoneurium, perineurium and epineurium) resulting in complete nerve transection (degree V).

**Table 1: Classification of peripheral nerve injuries Damaged tissue**

| | | |
|---|---|---|
| Neurapraxia | Degree I | Myelin sheath |
| Axonotmesis | Degree II | Myelin sheath, axon |
| Neurotmesis | Degree III | Myelin sheath, axon, endoneurium |
| | Degree IV | Myelin sheath, axon, endoneurium, perineurium |
| Degree V | Myelin sheath, axon, endoneurium, perineurium, epineurium | |

The present invention therefore also relates to the use of the present cell constructs in neurapraxia, axonotmesis, and neurotmesis, preferably in neurotmesis or for use the treatment of Degree I to V injuries, preferably of Degree III to V injuries. Accordingly, the present invention also relates to methods for treating these diseases, syndromes and injuries by administering an effective amount of these cell constructs to a patient in need thereof, e.g. by transplanting such a cell construct to the injured or diseased nerve region.

The present invention is further exemplified by the following examples and the figures, yet without being limited thereto.

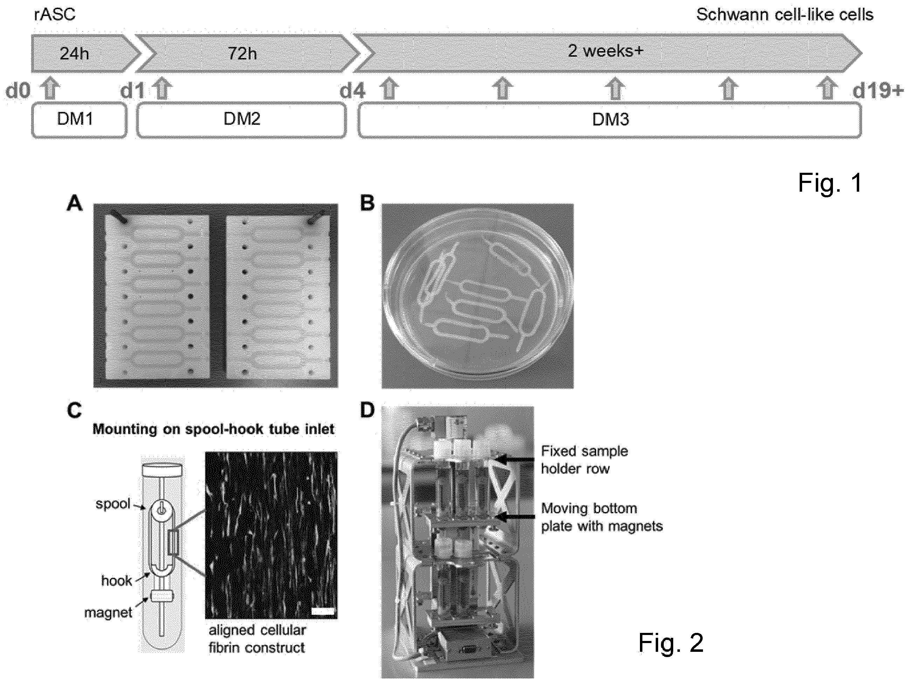

Fig. 1 shows that the differentiation of rat adipose-derived stem cells into Schwann cell-like cells requires the use of three different differentiation media. Cells are first incubated in growth medium supplemented with 1 mM β-mercaptoethanol for 24 hours (DM1), followed by preconditioning in growth medium containing 35 ng/µl all-trans retinoic acid for 72 hours (DM2). Afterwards, cells are cultivated in medium containing bFGF, PDGF, heregulin β1 as well as forskolin for at least 14 days (DM3), with media exchanges every third day. (DM - differentiation medium);
Fig. 2 shows the set-up of the MagneTissue bioreactor system. A: Ring-shaped fibrin hydrogels are cast using polyoxymethylene (POM) molds. B: Polymerized fibrin rings after casting. C: Schematic of the spool-hook system onto which ring-shaped fibrin hydrogels are mounted for the MagneTissue bioreactor. A magnet is integrated into the hook. The bar bottom right in the picture of the aligned cellular fibrin construct indicates 100 µm. D: The MagneTissue bioreactor consists of a sample holder row and a bottom plate with integrated magnets that interact with the magnets of the hooks, thereby enabling stretching of the constructs via magnetic force transmission when the bottom plate is moved up and down through a connected stepper motor.

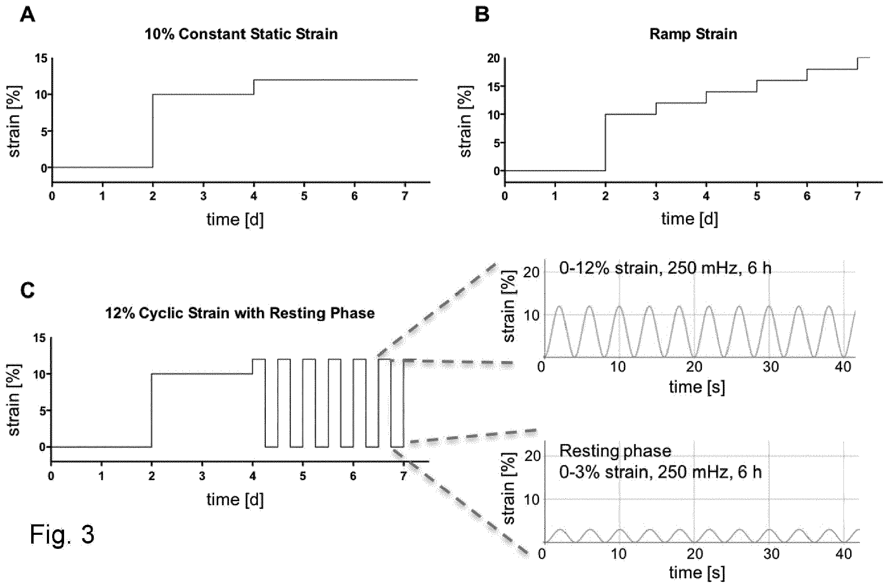

Fig. 3 shows static, cyclic and ramp strain regimes applied to Schwann cell-embedded fibrin hydrogels. A: Constant static strain of 10% was applied 48 hours after casting of fibrin rings and maintained for another 48 hours, followed by an increase to 12% constant static strain until day 7. B: In the ramp strain group, scaffolds were subjected to 10% constant static strain 48 hours after casting, which was followed by an increase of static strain by 2% per day, resulting in a maximum of 20% strain exposed to the constructs on day 7. C: The cyclic strain regime consisted of a combination of both, static and cyclic strain. 48 hours after casting, 10% constant static strain was first applied for 2 days, after which the strain regime was switched to 2 repetitions each day of 0-12% cyclic strain at 250 mHz for 6 hours and subsequently a resting phase at 0-3% cyclic strain at 250 mHz for 6 hours (see close-ups). This cyclic strain protocol was maintained until day 7.

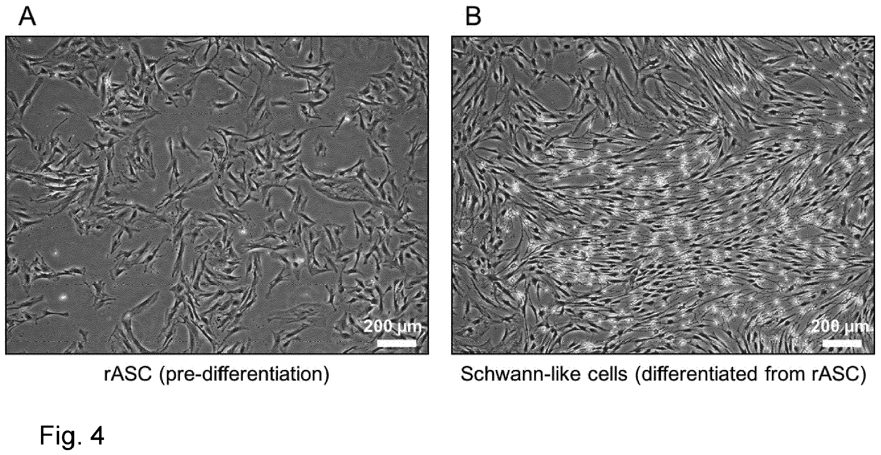

Fig. 4 shows the differentiation of rat adipose-derived stem cells (rASC) into Schwann cell-like cells. A: ASC were isolated from rat epididymal fat pads and show the typical flattened, spindle-shaped morphology. Cells were subsequently cultivated in Schwann cell differentiation medium containing a mix of different growth factors. B: After 20 days of differentiation, most cells have remodeled and obtained the typical Schwann cell morphology with a small and shiny cell body and long protrusions. Scale bars bottom right indicate 200 µm.

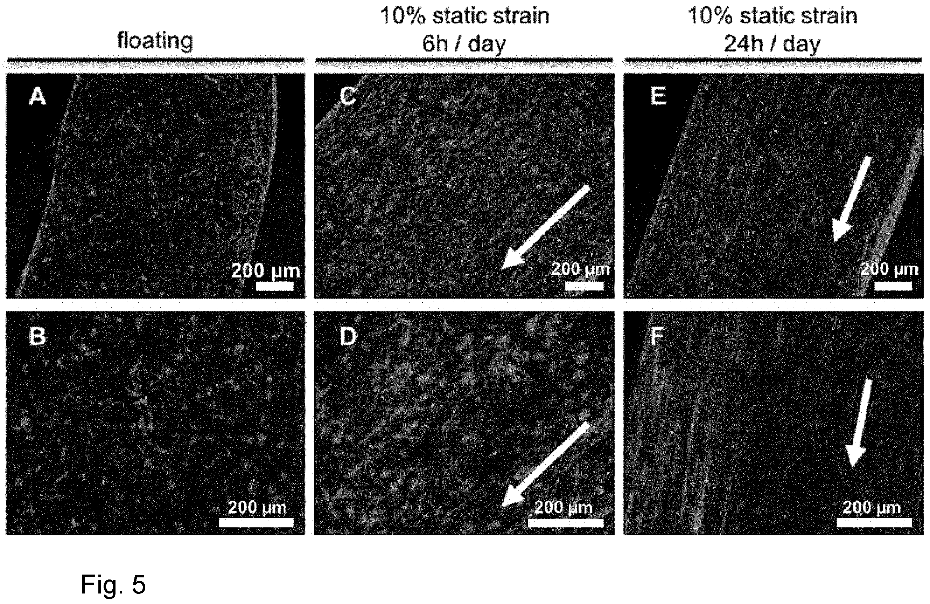

Fig. 5 shows that mechanical stimulation induces cellular alignment of putative Schwann cell-like cells. 250,000 cells were embedded in fibrin hydrogels and subjected to 10% static strain either constantly or for 6 hours a day, while floating rings served as controls. A, B: Cells cultured as floating controls oriented randomly inside the fibrin matrices. C, D: 10% static strain applied for 6 hours a day leads to minor alignment of putative Schwann cell-like cells along the axis of strain. E, F: In contrast, stimulation with 10% constant static strain generates highly aligned Schwann cell-like cell fibrin constructs, Cells were stained with phalloidin to visualize F-actin filaments. Arrows indicate the axis of strain. Scale bars bottom right indicate 200 µm.

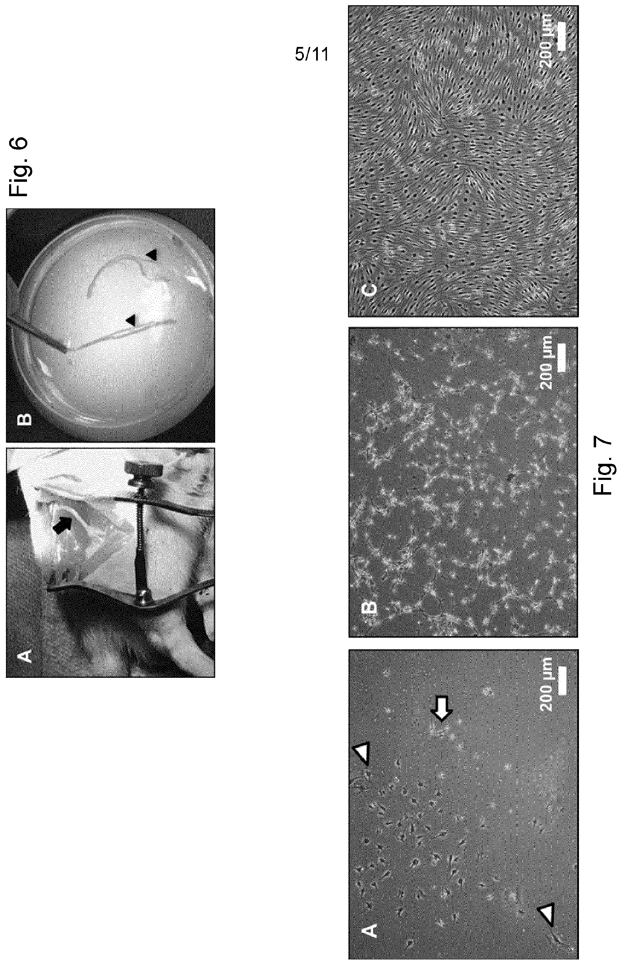

Fig. 6 shows the isolation of Schwann cells. A: Sciatic nerves were isolated from rat Sprague Dawley rats. The arrow indicates the exposed sciatic nerve. B: Removal of the epineurium is critical to minimize potential fibroblast contamination. After epineurial removal, the individual nerve fascicles become visible (arrowheads).
Fig. 7 shows the different stages during primary Schwann cell culture and morphologic characteristics. A: Brightfield images of Schwann cell culture 5 days after isolation, showing the presence of both fibroblasts (arrowhead) as well as Schwann cells (arrow). B: On day 15, fibroblasts are no longer present while Schwann cells are proliferating and show their typical bipolar morphology. C: At confluency, Schwann cells exhibit their characteristic swirling pattern. Scale bars bottom right indicate 200 µm.

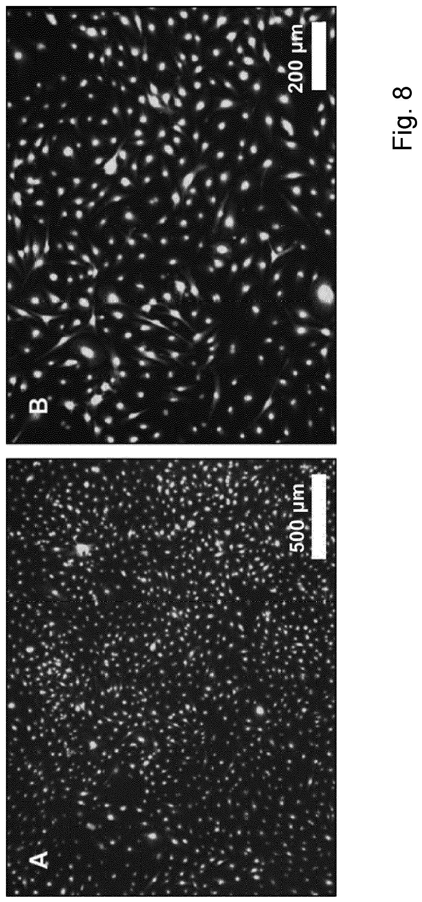

Fig. 8 shows isolated Schwann cells stained positively for the glial cell marker S100. A, B: Almost all cells obtained after Schwann cell isolation are stained positive for S100 (green), which is expressed both in the nuclei as well as in the cytoplasm. Note the different intensities of S100 expression among cells. Cells were counterstained with DAPI (blue) to visualize nuclei to distinguish between Schwann cells and S100 negative cells. Scale bars bottom right indicate 500 µm (A) and 200 µm (B).

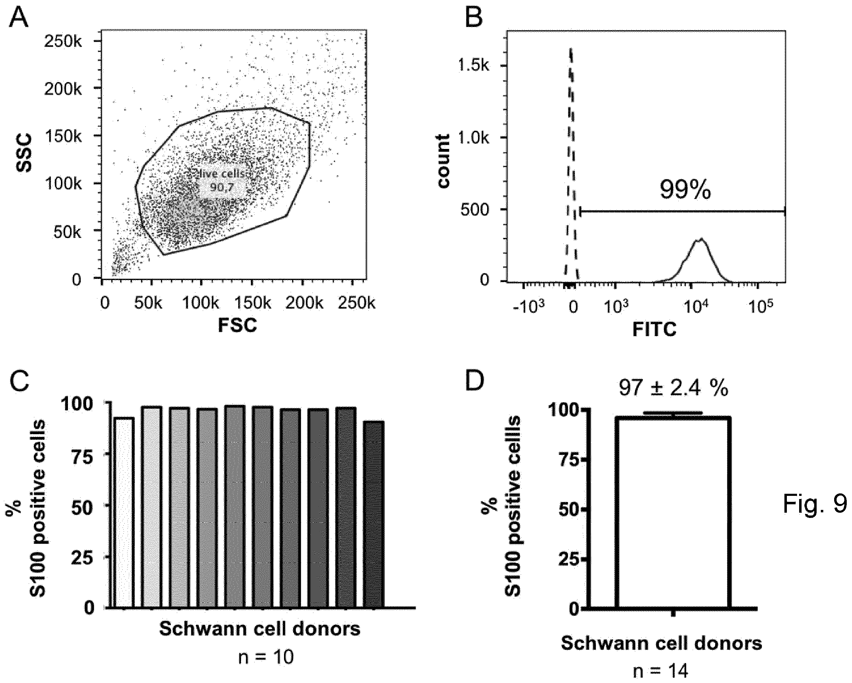

Fig. 9 shows that primary cell isolations from rat sciatic nerves achieved highly pure Schwann cell cultures as analysed by flow cytometry. A: Representative Schwann cell flow cytometry dot plot with the gated population ("live cells 90.7"). The gate was set to include most viable cells, while excluding cell debris, dead cells, and duplets. This gate was used for analysis of S100 positive live cells. B: Representative flow cytometry histogram of S100-stained cells, showing that 99% of live cells expressed S100. Dashed line shows secondary antibody control staining, solid line shows specific S100 staining. C: Schwann cell purities obtained from 10 individual donors show that all isolations yielded purities of over 90%. D: Average S100 expression of all Schwann cell donors revealed that high Schwann cell purities of 97 ± 2.4% were obtained.

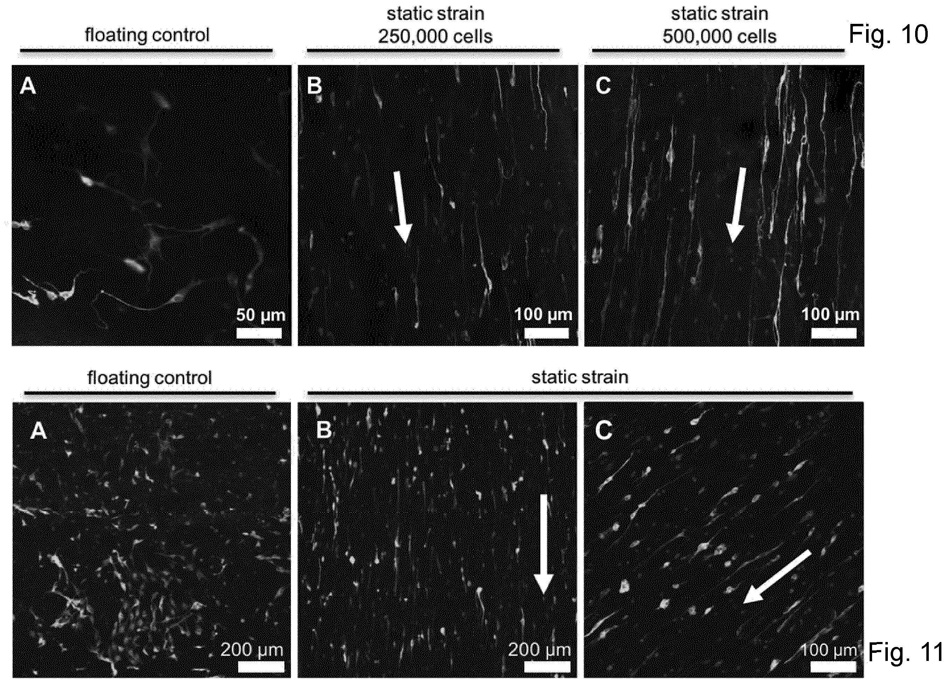

Fig. 10 shows that application of 10% static strain induced both elongation and alignment of Schwann cells. 250,000 and 500,000 cells were embedded in ring-shaped fibrin hydrogels. A: Cells cultivated as floating controls were randomly distributed inside fibrin hydrogels. B, C: Conversely, application of 10% constant static strain induced alignment of Schwann cells. The effect of mechanical stimulation on cellular behaviour was assessed by immunolabeling with the glial marker S100 (green) together with a DAPI counterstain (blue) to visualize nuclei. Scale bars bottom right indicate 50 µm (A) and 100 (B, C) µm.
Fig. 11 shows that static strain induces adequate formation of aligned bands of Büngner-like structures in fibrin matrices containing 2 x 10⁶ cells. A: Cells cultivated as floating controls oriented randomly inside fibrin hydrogels. B, C: In contrast, 10% static mechanical stimulation generated highly aligned Schwann cell constructs. The effect of mechanical stimulation on cellular behaviour was assessed by immunolabeling with the glial marker S100 (green) together with a DAPI counterstain (blue) to visualize nuclei. Scale bars bottom right indicate 200 µm (A, B) and 100 µm (C) .

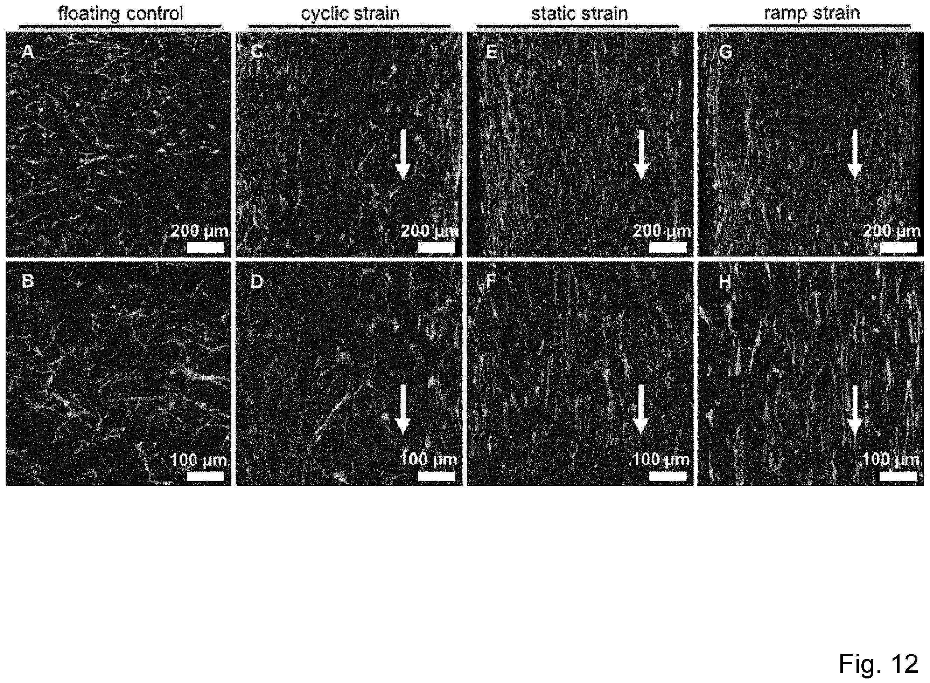

Figure 12 shows that different strain types strongly influence the degree of Schwann cell alignment. 3 x 10⁶ cells were embedded into fibrin hydrogels and after a resting phase of 48 hours subjected to either 10% static strain, which was increased to either 12% on day 4 (static strain) or switched to alternating phases of 0-12% cyclic strain followed by 0-3% cyclic strain for 6 hours each per day (cyclic strain). Additionally, a ramp strain regime was applied, increasing the static strain by 2% each day, finally resulting in 20% strain on day 7 (ramp strain). Rings cultivated floating in growth medium served as controls. While both, static and ramp strain induced pronounced alignment of Schwann cells along the axis of strain, application of cyclic strain only led to minor alignment. Cells were stained for the glial marker S100 (green) and nuclei were counterstained with DAPI. Arrows indicate the axis of strain. Scale bars bottom right indicate 200 µm (A, C, E, G) and 100 µm (B, D, F, H).

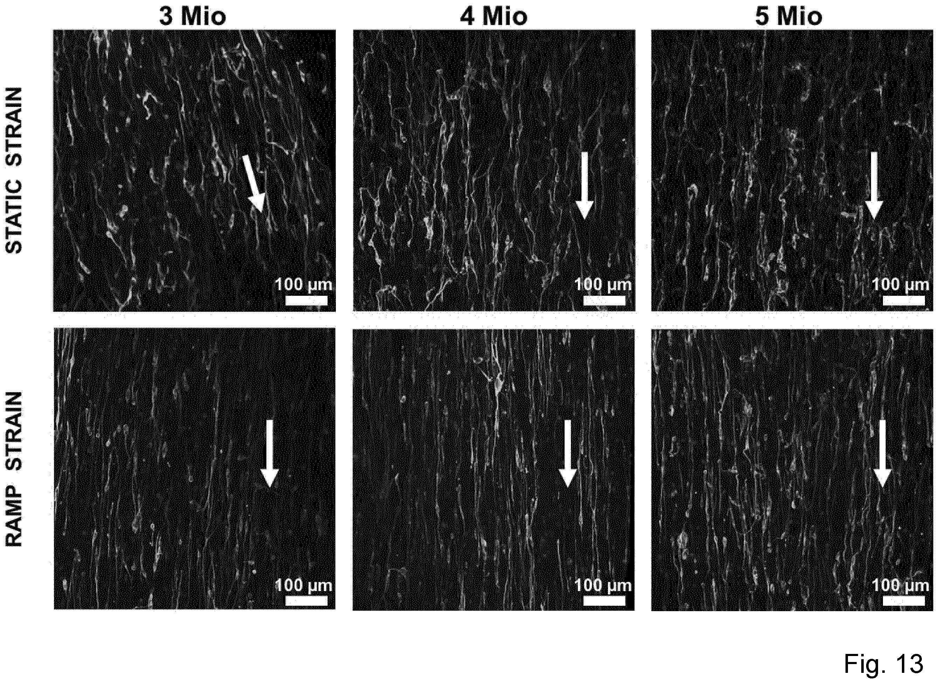

Figure 13 shows that superior alignment of embedded Schwann cells can be achieved with ramp strain treatment compared to static strain. 3 x 10⁶, 4 x 10⁶, or 5 x 10⁶ cells were embedded into fibrin hydrogels and after a resting phase of 48 hours subjected to either 10% static strain, which was increased to either 12% on day 4 (static strain) or a ramp strain regime by increasing the static strain by 2% each day, finally resulting in 20% strain on day 7 (ramp strain). The most appropriate band of Büngner-like constructs were achieved upon ramp strain treatment of 4 x 10⁶ Schwann cells embedded in fibrin hydrogels. Cells were stained for the glial marker S100 (green) and nuclei were counterstained with DAPI. Arrows indicate the axis of strain. Scale bars bottom right indicate 100 µm.

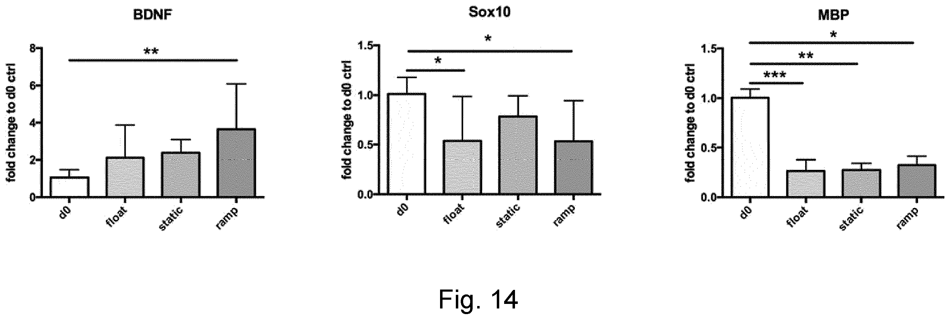

Figure 14 shows the influence of mechanical stimulation on gene expression in highly aligned Schwann cells as analysed by qPCR. The most prominent changes were present in the expression of the pro-regenerative marker BDNF, which was upregulated in all groups on day 7 compared to day 0, but with the strongest upregulation by approximately 3.6-fold seen in constructs subjected to ramp strain. Concomitantly, the expression of myelin-associated genes Sox10 and MBP was downregulated after 7 days of cultivation. Data are shown as bar graphs with mean + SD (floating, ramp: n = 10 from 4 independent experiments; static: n = 6 from 2 independent experiments). Statistical analysis was performed using Kruskal-Wallis test and Dunn's multiple comparisons test (*p<0.05, **p<0-01. ***p<0.001).

### Examples:

In the present examples, the present invention is illustrated by investigating a preferred embodiment of the present invention: a fibrin-based construct. The present examples investigate the generation of fibrin hydrogels with aligned Schwann cells applied either alone or as a novel intraluminal filler for nerve guidance conduits to speed up peripheral nerve regeneration. Therefore, two different cell types were compared: on the one hand, Schwann cells were directly isolated from rat sciatic nerves, while on the other hand, rat ASC were isolated and subsequently differentiated towards the Schwann cell lineage. To generate the peripheral nerve constructs, cells were embedded in ring-shaped fibrin hydrogels and different seeding densities ranging from 250,000 to 5,000,000 cells were compared. Based on the results of these preliminary experiments, the following experiments focused on the use of primary Schwann cells. Finally, the impact of different strain regimes (static vs. cyclic vs. ramp strain using the MagneTissue bioreactor) on Schwann cell alignment as well as gene expression was evaluated.

### Materials and Methods

If not stated otherwise, all reagents were purchased from Sigma Aldrich (Vienna, Austria).

### 1. Schwann cell isolation and culture

### 1.1 Preparation of culture surfaces

To improve Schwann cell adhesion and proliferation, culture surfaces were coated with poly-L-lysine and laminin. Therefore, the required culture plates were incubated with 0.01% poly-L-lysine solution for 10 min at room temperature. After incubation, the solution was collected for reuse and surfaces were washed twice with distilled water. After drying, culture surfaces were incubated with 0.01% (v/v) laminin diluted in phosphate buffered saline (PBS; Lonza) for at least 30 min at 37°C. Afterwards, culture surfaces were washed twice with PBS prior to cell seeding.

### 1.2 Sciatic nerve dissociation and seeding

Rat sciatic nerves isolated from male Sprague Dawley rats were gratefully received from the Ludwig Boltzmann Institute of Experimental and Clinical Traumatology (Vienna, Austria) and the Medical University of Vienna (Center of Biomedical Research, Vienna, Austria). To isolate Schwann cells, the epineurial connective tissue was first removed using fine-pointed forceps under a stereo microscope, so that nerve fascicles with their characteristic white stripes can be observed. These fascicles were then teased with a needle and enzymatically digested in 10 ml of 0.05% collagenase type I solution (Table 2) per pair of nerves at 37°C and 5% CO₂ for 60-90 min, depending on tissue dissociation. At the end of collagenase incubation, residual non-digested tissue was mechanically dissociated using a glass Pasteur pipette and the cell suspension was filtered through a 70 µm cell strainer. Cell suspension was centrifuged at 400 x g for 6 min and the cell pellet was resuspended in Dulbecco's Modified Eagle's Medium (DMEM; Lonza) containing 10% fetal calf serum (FCS; GE Healthcare), 1% Penicillin/Streptomycin (P/S) and 1% L-glutamine. After centrifugation at 400 x g for 6 min, the cell pellet was resuspended in Schwann cell isolation medium, consisting of DMEM D-valine (Cell Culture Technologies) supplemented with 10% FCS, 1% P/S, 1% L-Glutamine, 250 ng/ml Amphotericin B, 10 µg/ml bovine pituitary extract (PEX), 1x N2 supplement (ThermoFisher Scientific), 10 ng/ml recombinant human heregulin β1 (PeproTech)and 2 µM forskolin. Eventually, the cell suspension obtained from 1 pair of sciatic nerves was seeded into two 6-wells that have been sequentially coated with poly-L-lysine and laminin and incubated in a humidified atmosphere at 37°C and 5% CO₂. This primary cell culture was referred to as passage 0 (p0). After 7 days, 1 ml of fresh Schwann cell culture medium was added and cells were incubated 3 to 4 more days. On day 10 or 11, half of the medium was replaced with fresh Schwann cell medium, followed by 50% partial media exchanges every 2 to 3 days until confluence was reached.

**Table 2: Preparation of Collagenase I solution for enzymatic digestion Collagenase solution**

| | |
|---|---|
| DMEM, serum-free (Lonza) | 10 ml |
| 0.05% Collagenase I (Sigma Aldrich) | 5 mg |
| 1% P/S (Lonza) | 100 µl |
| 250 ng/ml Amphotericin B (Lonza) | 10 µl |

### 1.3 Schwann cell expansion

Primary Schwann cell cultures typically reached confluence from day 10 onwards. For further expansion, cells were enzymatically detached using trypsin, centrifuged at 400 x g for 6 min, the cell pellet was resuspended in fresh Schwann cell culture medium (DMEM D-valine supplemented with 10% FCS, 1% P/S, 1% L-Glutamine, 10 ng/ml recombinant human heregulin β1 and 2 µM forskolin) and cells were seeded in a 1:2 dilution onto poly-L-lysine-coated culture plates. A splitting ratio of 1:2 to 1:4 was maintained until a sufficient cell number was reached for Schwann cell characterization and mechanical stimulation experiments.

### 1.4 Freezing and thawing

For each successful isolation, Schwann cells at approximately 80% confluence were harvested for cryopreservation. Cells were washed twice with PBS and detached with trypsin. Detached cells were pelleted by centrifugation at 400 x g for 6 min and subsequently frozen in 50 µl dimethyl sulfoxide (DMSO) and 950 µl FCS.

For thawing of frozen cells, 6 ml medium was pre-warmed at 37°C, 5% CO₂ in 10 cm dishes coated with poly-L-lysine and laminin. The cryo vial was thawed in a water bath at 37°C and cell suspension was immediately transferred to a Falcon tube containing 4 ml pre-warmed medium. After centrifugation at 400 x g for 6 min to remove DMSO, cells were resuspended in growth medium and transferred to the prepared 10 cm dishes to be cultivated in a humidified atmosphere at 37°C and 5% CO₂.

### 2. Isolation of rat adipose-derived stem cells

Rat adipose-derived stem cells (rASC) were isolated from epididymal fat pads received from the Ludwig Boltzmann Institute of Experimental and Clinical Traumatology (Vienna, Austria). Fat tissue was washed twice with PBS containing 1% PS and 250 ng/µl Amphotericin B and cut into small pieces using a scalpel. Subsequently, minced epididymal fat tissue was enzymatically digested using 20 ml PBS supplemented with 1.5% Collagenase Type I, 2% bovine serum albumin (BSA) and 25 mM HEPES for approximately 3 hours at 37°C with continuous shaking. After filtration through a 100 µm cell strainer to remove any residual undigested tissue, the cell suspension was centrifuged at 400 x g for 7 minutes. The supernatant, containing mature adipocytes, was discarded and the pellet was washed with PBS, followed by centrifugation for 7 minutes at 400 x g. The pellet, containing rASC, was resuspended in growth medium (DMEM supplemented with 10% FCS, 1% P/S and 1% L-glutamine) and cells were seeded into a T175 flask. Medium was exchanged every 2-3 days and cells were split when reaching approximately 80% confluence.

### 3. Differentiation of rat adipose-derived stem cells into Schwann cell-like cells

Rat ASC were differentiated into Schwann cell-like cells as described elsewhere (Schuh et al., Cells Tissues Organs 200 (2014), 287-299; Dezawa et al., Eur. J. Neurosci. 14 (2001), 1771-1776). Briefly, rASC were seeded into T75 flasks at a density of 500 cells/cm² in growth medium. After 48 hours, medium was discarded and incubated in growth medium supplemented with 1 mM β-mercaptoethanol for 24 hours (Figure 1; DM1). Then, the cells were washed three times with PBS and incubated in growth medium supplemented with 35 ng/ml all-trans retinoic acid for 72 hours (Figure 1; DM2). Afterwards, cells were again washed three times with PBS and cultured in Schwann cell-like differentiation medium, consisting of growth medium supplemented with 10 ng/ml recombinant rat basic fibroblast growth factor (bFGF; PeproTech), 5 ng/ml recombinant murine platelet-derived growth factor (PDGF; PeproTech), 200 ng/ml recombinant human heregulin β1 (PeproTech) and 14 µM forskolin (Figure 1; DM3). Schwann cell-like differentiation medium was exchanged every third day and cells were split when reaching confluence.

### 4. Schwann cell characterization

To evaluate isolation efficiency and verify Schwann cell identity, isolated and differentiated cells were analysed by flow cytometry as well as immunofluorescence stainings.

### 4.1 Flow Cytometry

For flow cytometric analysis, cells were detached with accutase to preserve surface marker expression, centrifuged at 400 x g for 6 min and subsequently fixed in 1% formaldehyde (PFA; Roth) in PBS for 15 min on ice. Afterwards, 2 ml of PBS were added, cells were pelleted by centrifugation at 400 x g for 6 min and resuspended in 1% (w/v) BSA in PBS, hereafter referred to as PBS/BSA. Cells were then either stored at 4°C or stained immediately for flow cytometry.

For immunostaining, 100 µl of cell suspension were transferred to each flow cytometry tube. For staining with the intracellular marker S100, cell membranes were first permeabilized by adding 900 µl methanol dropwise to the cell suspension under gentle vortexing and subsequent incubation on ice for 30 min. Permeabilized cell suspensions were washed with 1 ml PBS/BSA and resuspended in 100 µl PBS/BSA. For labelling, rabbit polyclonal S100 primary antibody was added 1:400, cells were vortexed shortly and incubated on ice for 30 min. To remove unbound antibodies, 1 ml PBS/BSA, was added and cell suspensions were centrifuged at 400 x g for 6 min. The supernatants were carefully discarded and the washing step was repeated with 1 ml PBS/BSA. After decanting the supernatants, goat anti-rabbit AlexaFluor 488 secondary antibody diluted 1:400 in 100 µl PBS/BSA was added, tubes were shortly vortexed and incubated for 30 min on ice in the dark to avoid bleaching of the fluorophores. Following secondary antibody incubation, cells were washed with 1 ml PBS/BSA and centrifuged at 400 x g for 6 min. Supernatants were discarded and the washing step was repeated to remove residual unbound antibodies. Cells were then resuspended in 300 µl PBS/BSA and analysed with a BD FACS Canto II (Becton Dickson Biosciences, Franklyn, USA) flow cytometer. Unstained cells as well as cells stained only with the secondary antibody served as controls. Data were analysed using FlowJo Version 10.4.2 (Tree Star, Ashland, USA).

### 4.2 Immunofluorescence

In addition to flow cytometry analysis, Schwann cells cultured in 12-well plates (CytoOne) coated with PLL and laminin were fixed with 4% PFA for 15 min at room temperature, washed twice with PBS for 5 min each and subsequently blocked and permeabilized in blocking solution, consisting of PBS supplemented with 1% (w/v) BSA, 5% (v/v) FCS and 0.2% (v/v) Triton X-100 for 1 hour on an orbital shaker. Afterwards, cells were washed twice with PBS and incubated for 1 hour with rabbit polyclonal S100 primary antibody diluted 1:200 in blocking solution. Prior to incubation with goat anti-rabbit AlexaFluor 488 secondary antibody (1:400 in blocking solution), cells were washed 3 times with PBS in order to remove unbound primary antibody. After secondary antibody incubation for 1 hour, cells were washed three times with PBS and subsequently counterstained for approximately 5 min with 4',6-diamidino-2-phenylindole (DAPI) diluted 1:1000 in blocking solution. Cells were washed another time with PBS and visualized on a Leica DMI 6000b epifluorescence microscope (Leica Microsystems, Germany).

### 5. Generation of ring-shaped Schwann cell-enclosed fibrin hydrogels

In order to assess the impact of mechanical stimulation on Schwann cell alignment and putative phenotypic changes, cells were embedded in ring-shaped fibrin hydrogels using the Tissucol Duo fibrin kit (Baxter AG, Vienna, Austria). Fibrinogen was diluted in Schwann cell medium to a working concentration of 40 mg/ml. Thrombin was diluted to a concentration of 4 IU/ml in 40 mM calcium chloride, followed by further dilution in Schwann cell suspension to the working concentration of 1.25 IU/ml. Fibrin scaffolds were cast using ring-shaped polyoxymethylene (POM) molds (Figure 2A) by mixing 250 µl of the diluted fibrinogen solution with 250 µl of the thrombin/cell suspension solution, resulting in final concentrations of 20 mg/ml fibrin and 0.625 IU/ml thrombin. After polymerization for one hour at 37°C, fibrin rings were retrieved from the molds (Figure 2B), mounted on the MagneTissue spool/hook system and cultured in Snap-Cap tubes (Figure 2C) containing 9 ml Schwann cell medium supplemented with 100 KIU/ml aprotinin. Rings floating in growth medium inside Snap-Cap tubes (BD Biosciences) served as controls. Medium was exchanged every two to three days.

### 5.1 Mechanical stimulation of Schwann cell hydrogels using the MagneTissue bioreactor

Mechanical stimulation was applied to 3D cell-enclosed fibrin hydrogels using the MagneTissue bioreactor system via magnetic force transmission (Heher et al., Acta Biomater. 24 (2015), 251-265). Therefore, scaffolds are mounted onto a spool-hook system and placed into SnapCap tubes as described above (Figure 2C). The bioreactor consists of an upper sample holder row and a bottom plate with integrated magnets (Figure 2D), which exert an attraction force when interacting with the magnets integrated into the hooks. Mechanical stimulation is exerted via magnetic force transmission by movement of this bottom plate via a stepper motor (Heher et al., 2015).

For mechanical stimulation, three different strain regimes were employed in addition to a control group consisting of rings floating in the tubes (Figure 3). During the first two days after ring casting, all scaffolds were cultivated unstrained to provide a recovery time from the casting procedure. Starting from day 2, three different strain regimes were compared. One group was subjected to 10% constant static strain for 48 hours, which was increased to 12% for the remaining 72 hours (Figure 3A). The second stimulation protocol consisted of a ramp strain, where static strain was increased for 2% every day, starting at 10% static strain on day 2 and ending at a maximum of 20% static strain on day 7 (Figure 3B). In the third group, 10% constant static strain was first applied for 48 hours starting on day 2, followed by a switch to a more dynamic protocol of two repetitions of (i) 0-12% cyclic strain for 6 hours at 250 mHz and (ii) 6 hours "resting phase" at 0-3% cyclic strain (250 mHz) per day until day 7 (Figure 3C) .

### 6. Analysis of the impact of mechanical stimulation on Schwann cell behaviour

To evaluate the impact of mechanical stimulation on gene expression as well as cellular alignment, qPCR analyses and immunofluorescence (IF) stainings were performed.

### 6.1 Immunofluorescence

To visualize Schwann cell alignment after mechanical stimulation, fibrin rings were fixed in 4% PFA for either two hours at room temperature or at 4°C overnight. Afterwards, scaffolds were washed three times with distilled water, incubated in 50% ethanol for 30 min and then stored in 70% ethanol at 4°C for a maximum of 1 week until immunofluorescence staining was performed. Prior to staining, ethanol was removed and scaffolds were washed twice with PBS. Afterwards, scaffolds were first incubated in 0.1% (v/v) Triton X-100 in Tris-buffered saline (TBS/T) and then in PBS containing 0.1% (v/v) Triton X-100 (PBS/T) for 15 min each at room temperature to permeabilize the cells. Subsequently, scaffolds were blocked using PBS supplemented with 1% (w/v) BSA, 5% (v/v) FCS and 0.2% (v/v) Triton X-100 for 1 hour at room temperature. Afterwards, cells were washed three times with PBS/T and incubated in a 1:400 dilution of S100 in PBS/T-1% BSA at 4°C in Eppendorf tubes on an orbital shaker overnight. The next day, scaffolds were washed three times with PBS/T and incubated with an AlexaFluor 488 goat anti-rabbit (Invitrogen, California, USA) secondary antibody diluted 1:400 in PBS/T-1% BSA at 37°C for one hour. After three washing steps with PBS followed by another washing step with PBS/T, scaffolds were incubated in 0.1 M glycine for 5 min to reduce the background. For visualization of nuclei, cells were counterstained with a 1:1000 dilution of DAPI in PBS/T-BSA for 10 min in the dark. Scaffolds were washed with PBS and subsequently with water to remove salt residues, and embedded in Mowiol mounting medium (Table 3) on glass cover slides (VWR, Vienna, Austria) to preserve the immunofluorescence staining. Slides were analysed on a Zeiss LSM700 confocal microscope and stored at 4°C in the dark.

**Table 3: Formulation of mowiol mounting medium for preservation of immunofluorescence stained scaffolds on glass slides. Mowiol mounting medium**

| | | |
|---|---|---|
| 6 | g | glycine |
| 2.6 | g | Mowiol 4-88 |
| 6 | ml | ddH₂O |
| 12 | ml | Tris/HCl pH 8.5 |

### 6.2 Trizol isolation of RNA

For isolation of RNA, fibrin rings were snap-frozen in liquid nitrogen and either stored at -80°C or processed immediately. Therefore, frozen rings were washed three times with PBS, subsequently snap frozen in opened 2 ml Eppendorf tubes in liquid nitrogen and immediately crunched using special tweezers for homogenization. Afterwards, 1 ml peqGOLD TriFast (hereafter referred to as trizol; VWR, Vienna, Austria) was added and tubes were vortexed for 10 min. After 15 min of incubation at room temperature, chloroform was added 1:5 of initial trizol used, tubes were shook vigorously for 15 seconds and incubated for approximately 10 min at room temperature until density gradient formation was observable. Tubes were then centrifuged for 15 min at 12,000 x g and 4°C, and the upper aqueous phase containing RNA was transferred to fresh Eppendorf tubes with caution to not disturb the interphase and phenol-phase. Afterwards, it was either proceeded immediately with RNA isolation, or samples were frozen at -20°C.

To precipitate RNA in the aqueous phase, isopropanol was added 1:2 of initial trizol used and samples were incubated on ice for 15 min. After centrifugation at 12,000 x g for 10 min, the RNA pellet was washed with 75% ethanol, vortexed shortly and centrifuged at 12,000 x g for 10 min. The supernatant was decanted, the washing step was repeated and RNA pellets were allowed to air-dry in open and inverted tubes for 20 min at room temperature. Eventually, the RNA pellet was dissolved in 15 µl RNase-free water and concentration as well as A260/A280 ratio determined on a NanoDrop photometer (Thermo Fischer, Vienna, Austria).

### 6.3 qPCR

Reverse transcription of isolated mRNA into cDNA was performed using the EasyScript cDNA synthesis kit (abm, Richmond, Canada). A master mix of all reagents (Table 4) was prepared for all samples and the amount of RNA used per reaction was adjusted to the sample with the lowest concentration measured, using at least 140 µg RNA per reaction. 7.5 µl master mix per reaction were pipetted into 0.2 ml PCR reaction tubes and 12.5 µl RNA diluted in RNase-free dH₂O were added to reach a final volume of 20 µl per reaction. cDNA synthesis was performed using an Eppendorf PCR thermocycler with the following conditions:
- 25°C for 10 min for primer extension
- 42°C for 60 min for reverse transcription
- 85°C for 5 min for inactivation of reverse transcriptase
- 4°C until further processing.

**Table 4: Composition of cDNA synthesis master mix per reaction.**

| | | |
|---|---|---|
| Reagent | | Volume per reaction [µl] |
| Master | 5 xRT buffer | 4 |
| Mix | Oligo(dT) [10 µM] | 1 |
| | dNTPs [10 mM] | 1 |
| | RNase Off Inhibitor [40 U/µl] | 0.5 |
| | Reverse Transcriptase | 1 |
| | RNA + RNase-free water | 12.5 |
| | Total volume | 20 |

For gene expression analysis via qPCR, all samples were analysed in triplicates, and non-template controls were included for each gene of interest. Template cDNA samples were diluted to a working concentration of 3.33 ng/µl to obtain a total amount of 10 ng cDNA per reaction, which was advanced to a 96-well PCR plate (StarLab, Hamburg, Germany). A master mix of all reagents was prepared per gene of interest, containing PerfeCTa SYBR Green FastMix (Quanta Biosciences, Gaithersburg, Maryland, USA), primers diluted to concentrations 400 nM (200 nM for the housekeeper) and RNase-free water (Table 5), and 17 µl were added to each reaction. qPCR was performed using a Stratagene Mx3005P cycler (Agilent, California, USA) with a normal-2-step thermal profile setup. Target genes normalized to the housekeeping gene glyceraldehyde-3-phosphate dehydrogenase (GAPDH) were compared to either floating control or 2D samples, and fold changes were calculated using the ΔΔC_{T} method. Primer sequences are shown in Table 6.

**Table 5: Composition of a qPCR reaction with the KAPA SYBR Fast master mix. Per reaction, 2 ng cDNA was used and primers were diluted to 200 or 400 nM.**

| Reagent | Primer concentration | | | |
|---|---|---|---|---|
| | 200 | nM | 400 | nM |
| PerfeCTa SYBR Green FastMix | 10 | µl | 10 | µl |
| Primer forward | 0.4 | µl | 0.8 | µl |
| Primer reverse | 0.4 | µl | 0.8 | µl |
| RNase-free dH₂O | 6.2 | µl | 5.4 | µl |
| cDNA [0.5 ng/µl] | 3 | µl | 3 | µl |

**Table 6: Primer sequences for qPCR. Primers were purchased from Microsynth (Switzerland) and used at a final concentration of 400 nM, except for GAPDH, which was used at 200 nM concentration.**

| Target gene | Forward (5'-3') | Reverse (5'-3') |
|---|---|---|
| BDNF | TCTACGAGACCAAGTGTAATCCCA | CTTATGAACCGCCAGCCAAT |
| Sox10 | TGCCAAAGCCCAGGTGAAGA | AGACTGAGGGAGGTGTAGGCGAT |
| | | |
| MBP | GAAGTCGCAGAGGACCCAAGA | CTGCCTCCGTAGCCAAATCC |
| GAPDH | CCGTATCGGACGCCTGGTTA | CCGTGGGTAGAGTCATACTGGAAC |

### 7. Statistical analysis

Statistical evaluation was performed using the GraphPad Prism 6 software (GraphPad Software Inc., San Diego, USA). First, Grubbs' outlier analysis was performed to identify and exclude potential outliers. Shapiro-Wilk normality test was used to assess whether data are normally distributed. Nonparametric data were analysed using Kruskal-Wallis's test with Dunn's multiple comparison test. Level of significance was set at p < 0.05.

### Results

### 1. ASC can be transdifferentiated towards a Schwann cell-like phenotype

Since ASC demonstrate numerous beneficial characteristics for cell-based therapies in tissue engineering and regenerative medicine, they have also gained immense interest as a popular source for clinical applications in peripheral nerve repair. These cells can not only be easily isolated from adipose tissue with great yields devoid of donor-site morbidity and expanded rapidly in vivo, but they are also immunoprivileged and can be differentiated towards multiple lineages, including the Schwann cell lineage. Hence, ever since the first report of successful transdifferentiation of mesenchymal stem cells into Schwann cell-like cells (Dezawa et al., 2001), the regenerative potential and clinical eligibility was exploited for treatment of peripheral nerve injuries.

In the examples performed for the present invention, ASC were isolated from rat epididymal fat pads and passage 2 cells were subsequently pre-conditioned in medium supplemented with 1 mM β-mercaptoethanol for 24 hours, followed by addition of 35 ng/ml all-trans retinoic acid for 72 hours. Differentiation was induced using medium supplemented with heregulin, forskolin, PDGF and bFGF for a minimum of 19 days (Figure 1). Prior to differentiation as well as after the pre-conditioning phase, ASC exhibited a flattened and fibroblast-like morphology (Figure 4A). Starting from day 7 after the initiation of differentiation, ASC started to progressively remodel their cytoskeleton and adopted an elongated bi- or tripolar morphology with a small but prominent cell body and long protrusions by day 20 (Figure 4B).

### 2. Mechanical stimulation induces alignment of putative

Schwann cell-like cells embedded in fibrin hydrogels One central approach in peripheral nerve tissue engineering is the generation of aligned constructs that can subsequently be used as guidance structures by regenerating axons to bridge the nerve gap. To analyse whether Schwann cell-like cells can be aligned, rat adipose-derived stem cells transdifferentiated into Schwann cell-like cells were embedded in fibrin hydrogels at a density of 250,000 cells per ring and subjected to either 10% static strain for 6 hours or 24 hours a day. Rings cultivated floating inside SnapCap tubes served as controls and were oriented randomly inside the fibrin matrix (Figure 5A, B). In contrast, especially cells exposed to 10% constant static strain clearly aligned along the axis of strain (Figure 5E and F). This effect was not as pronounced in scaffolds strained for only 6 hours a day, although minor alignment was observable in this group as well (Figure 5C and D). However, putative Schwann cell-like cells were stained with phalloidin, which visualizes F-actin filaments and does not confirm Schwann cell-like cell identity. Consequently, whether these cells truly are Schwann cell-like cells or cells de-differentiated to rASC remains elusive.

### 3. Achievement of highly pure Schwann cell cultures from freshly isolated adult rat tissue

Since primary Schwann cells are furthermore a more suitable cell type compared to Schwann cell-like cells to gain insight into the molecular processes of glial cells during peripheral nerve regeneration, Schwann cells were used for further mechanical stimulation experiments. To exploit the therapeutic potential of Schwann cells for peripheral nerve regeneration, the ability to isolate highly pure Schwann cells is of utmost importance.

In the examples of the present invention, Schwann cells were successfully isolated based on a recently published protocol (Kaewkhaw et al., Nat. Protoc. 7 (2012), 1994-2004). The most important component of the isolation procedure is the use of DMEM supplemented with D-valine, which is a non-essential amino acid and can be preferentially metabolized by Schwann cells via the enzyme D-amino acid oxidase (DAAO) into the essential amino acid L-valine required for cellular proliferation and survival. Fibroblasts, in contrast, do not have this ability and therefore die after a few days in culture (Kaewkhaw et al., 2012).

Schwann cells were isolated from rat adult sciatic nerves due to their large size and easy harvesting. The skin was incised along the thigh to the knee and sciatic nerves were carefully separated from the surrounding connective tissue and muscle (Figure 6A). The outermost layer of the nervous connective tissue, the epineurium, was removed meticulously (Figure 6B) as this is the major source of potential fibroblast contamination. After epineurial removal, the individual nerve fascicles became visible, and the tissue was enzymatically dissociated.

Typically, cellular outgrowth could be observed from day 5 on, with initially both fibroblasts (Figure 7A, arrowhead) and Schwann cells (Figure 7A, arrow) being present. Fibroblasts failed to proliferate and died over time, resulting in highly pure Schwann cell cultures (Figure 7B). The typical bipolar Schwann cell morphology is marked by a prominent but tiny and usually shiny cell body with two long protrusions, while fibroblasts display a flattened and polymorphic shape. When reaching confluency, cells exhibited a swirling pattern (Figure 7C), which is a characteristic of Schwann cell behaviour (Kaewkhaw et al., 2012).

To confirm Schwann cell identity after isolation, cells were immunolabeled with the glial marker S100, which is specific for Schwann cells but should not stain fibroblasts, and counterstained with DAPI to visualize nuclei. The vast majority of cells obtained after isolation were stained positively for S100, which was, however, expressed at different intensities and localized in both the nuclei as well as in the cytoplasm. Only few cells stained positive for DAPI only, indicating the presence of non-glial cells (Figure 8).

In addition to immunostaining of cells, Schwann cell purity and identity was quantitatively evaluated by flow cytometry. Figure 9A shows a representative flow cytometry dot plot. A gate was set to include the majority of live Schwann cells (here 90.7%), as determined by their size and granularity, but excluded cellular debris, dead cells and duplets. Events inside this gate were then further used for analysis of the S100 fluorescent staining. S100 flow cytometry histogram of gated cells displayed a rather broad fluorescence intensity profile (Figure 9B), indicating a highly differential S100 marker expression among Schwann cells. This, in turn, is in accordance with the results obtained from immunostaining (Figure 8). Remarkably, all Schwann cell isolations always yielded purities over 90% (Figure 9C), and on average, isolation efficiency resulted in Schwann cell purities of 97 ± 2.4% after several passages from initial Schwann cell isolation (Figure 9D).

### 4. Schwann cell numbers as well as the application of different strain regimes strongly influence the capacity to form bands of Büngner-like structures using mechanical stimulation

After verification of Schwann cell identity via flow cytometry, cells from all donors were expanded for mechanical stimulation experiments. Therefore, cells between passages 5 and 7 were embedded in ring-shaped fibrin hydrogels to create a 3D environment and subjected to tensile strain. In the first experiment, fibrin rings with 250,000 cells and 500,000 cells (Figure 10) or 2 x 10⁶ cells (Figure 11) were cast and subsequently subjected to 10% constant static strain or cultured as floating controls. Since the findings obtained with Schwann cell-like cells revealed that subjecting cells to constant static strain 24 hours a day without resting phases, this was the strain regime of choice for first mechanical stimulation experiments using primary Schwann cells. While floating control hydrogels showed random distribution of Schwann cells (Figure 10A; Figure 11A), cells subjected to static tensile strain aligned along the axis of strain and furthermore appeared elongated (Figure 10B, C; Figure 11B, C).

In addition to the static strain regime evaluated before, also a more dynamic stimulation protocol was used. In this cyclic strain regime, 3 x 10⁶ cells were first subjected to 10% constant static strain for 48 hours to induce alignment of both the fibrin hydrogels as well as of Schwann cells, and subsequently, stimulation was switched to 6 hours of 12% cyclic strain at 250 mHz followed by a resting phase of 3% cyclic strain for 6 hours each day. This cyclic protocol was repeated twice a day for a total of three days. Compared to cells exposed to 10% constant static strain, the cellular alignment obtained with the cyclic stimulation protocol was not as pronounced and defined, and cells furthermore did not appear elongated as is the case in the static group (Figure 12C-F). However, there still is minor alignment even in the cyclic strain group when compared with the floating control group (Figure 12A, B).

Moreover, a third tensile stress stimulation protocol was investigated. Since it was hypothesized that the effect of constant strain is no longer present once the fibrin hydrogels adapted to the stretch they are exposed to and become worn out, a ramp style of static strain was employed to ensure the sustained provision of mechanical stress to the scaffolds. Therefore, cells are subjected to 10% constant static strain starting on day 2, and the strain is subsequently increased by 2% per day, resulting in a maximum strain of 20% on day 7. Indeed, results showed slightly better alignment of Schwann cells along the axis of strain (Figure 12G, H) compared to the static strain group (Figure 12E, F). Furthermore, cells subjected to the ramp strain profile appeared more elongated than cells exposed to constant static strain. In addition to that, the use of 3 x 10⁶ Schwann cells resulted in improved capacity to form bands of Büngner-like structures than the lower cell numbers tested before (compare Figure 10 and 11).

Hence, we aimed to further evaluate if the formation of bands of Büngner-like structures, which could potentially guide axonal regrowth in vivo, can be further optimized when using even higher cell numbers. Consequently, cell numbers of 3 x 10⁶, 4 x 10⁶ and 5 x 10⁶ cells per scaffold were compared in further MagneTissue experiments to evaluate the optimal cell concentration required to appropriately resemble the formation of bands of Büngner-like structures (Figure 13). Based on these experiments, it was decided to use 4 x 10⁶ cells for all further experiments. Again, cells subjected to ramp strain treatment showed superior alignment along the axis of strain (Figure 13).

### 5. Impact of mechanical stimulation of primary Schwann cells on gene expression levels

qPCR analysis was performed to analyse potential effects of static and ramp strain on the expression of both, pro-regenerative as well as myelin-associated genes. GAPDH was used as a housekeeping gene. Expression of the myelin gene MBP was significantly downregulated in all experimental groups compared to d0 controls, while downregulation of Sox10 was only significant in constructs cultured as floating controls as well as constructs exposed to ramp strain. In contrast, the expression of the pro-regenerative marker was significantly upregulated (3.6-fold) only in samples subjected to ramp strain (Figure 14).

### Discussion

Since the currently available approaches to treat peripheral nerve injuries are limited and mostly unsatisfactory in terms of functional recovery, peripheral nerve tissue engineering has recently gained immense interest in the scientific community. Especially the establishment of nerve guidance conduits is intensively being investigated, aiming at improving their potential to regenerate larger nerve gaps and to ultimately surpass autologous nerve grafts, which are the current gold standard. The present invention aims to provide novel peripheral nerve constructs consisting of aligned Schwann cell-embedded fibrin matrices using mechanical stimulation. Therefore, the impact of different strain parameters on Schwann cell alignment and gene expression was evaluated. These highly aligned Schwann cell constructs pose novel intraluminal fillers for NGCs to potentially speed up peripheral nerve regeneration by providing guidance cues for regenerating axons. The present invention, for the first time, provides aligned Schwann cell constructs via direct mechanical stimulation of the cells themselves embedded in matrices.

Several studies investigated the potential of different cell types inside NGCs to improve regeneration of nerve lesions, including mainly primary Schwann cells, mesenchymal stem cells and Schwann cell-like cells. Since the use of ASC presents several advantages in certain embodiments, such as simple harvesting of ASC from fat tissue, high proliferation rates in vitro and their immunoprivilege due to lack of MHCII expression, it is not surprising that their differentiation potential into Schwann cell-like cells is currently being exploited for peripheral nerve regeneration approaches. Therefore, the applicability of Schwann cell-like cells for the generation of aligned fibrin scaffolds was investigated. For this purpose, ASC isolated from rat epididymal fat were transdifferentiated into Schwann cell-like cells using three different differentiation media (Figure 1). Differentiation into Schwann cell-like cells was characterized by a transformation from the flattened polymorphic shape of rASC (Figure 4A) into an elongated, mostly bipolar morphology characteristic of Schwann cells (Figure 4B). For mechanical stimulation experiments using the MagneTissue bioreactor, 250,000 Schwann cell-like cells were embedded in ring-shaped fibrin hydrogels and subjected to 10% static strain either constantly, or for 6 hours a day. Additionally, scaffolds floating in growth medium served as controls. While cells cultivated as floating controls oriented randomly inside the fibrin matrices, especially cells subjected to 10% constant static strain showed a high degree of alignment along the axis of strain (Figure 5). However, one issue of this finding is that cells inside the fibrin scaffolds were not stained with a Schwann cell-specific marker, such as S100, but rather with phalloidin, which visualizes a cell's F-actin filaments.

In order to exploit the therapeutic potential of Schwann cells, isolation of highly pure Schwann cell cultures from adult tissue and efficient expansion in vitro is important. Since co-isolation of fibroblasts, which rapidly outgrow slowly proliferating Schwann cells, is a major obstacle, a popular approach to eliminate these cells is the use of antimitotic agents, such as cytosine arabinoside. However, these agents are not very selective and consequently also impair Schwann cell proliferation, resulting in low yields of Schwann cells. In the examples of the present invention, based on the protocol published by Kaewkhaw et al., 2012, DMEM formulated with D-valine was used instead of L-valine. Being an essential amino acid, L-valine is usually required by most cells for proliferation and survival. Schwann cells, however, have the ability to metabolize D-valine via the enzyme DAAO into L-valine, while fibroblasts do not have this ability and die after a few days in culture (Figure 7). This way, outstanding Schwann cell purities of 96 ± 2.38% could be obtained as quantified by S100 flow cytometry analysis (Figure 9).

Since it has been reported before that moderate tensile stress promotes peripheral nerve, it was investigated whether aligned Schwann cells resembling the formation of bands of Büngner can be obtained using mechanical stimulation, with the ultimate goal to assess whether these constructs are capable to accelerate the regenerative process taking place upon implantation. Therefore, isolated primary Schwann cells were expanded and embedded in fibrin hydrogels at different densities for mechanical stimulation experiments using the MagneTissue bioreactor system. In particular, the effect of three different strain protocols on Schwann cell alignment and gene expression was assessed. Based on the findings obtained in experiments conducted with Schwann cell-like cells, it was decided to expose the cells to constant static strain in two of these protocols, as constant strain resulted in superior alignment compared to scaffolds strained for only 6 hours a day (Figure 5). All these stimulation protocols started with a resting phase during the first two days, in which scaffolds were kept at 0% strain. In the constant static strain regime, cells were subsequently strained at 10% constant static strain for 2 days, followed by an increase to 12% strain until day 7 (Figure 3A). In the second protocol, cells were also first subjected to 10% constant static strain for 24 hours, followed by an increase in strain by 2% each day, reaching a final stain of 20% on day 7 (Figure 3B). The third protocol consisted of an initial phase of 10% constant static strain, after which the stimulation was switched to a more dynamic protocol of 2 repetitions of 0-12% cyclic strain at 250 mHz for 6 hours, followed by a resting phase at 0-3% cyclic strain per day (Figure 3C).

In the examples of the present invention, it was shown that application of constant static strain promoted alignment of Schwann cells in fibrin matrices using as little as 250,000 cells per scaffold (Figure 10). Nevertheless, cell densities of 250,000 and 500,000 cells may not be sufficient to induce the formation of bands of Büngner-like structures, whereas fibrin constructs prepared with a minimum of 3 x 10⁶ cells produced Schwann cell tracks of adequate densities and lengths (Figure 12). Furthermore, Schwann cells seemed to elongate in length and furthermore fuse with neighbouring Schwann cells, thereby forming continuous cellular tracks that could potentially serve as axonal guidance cues (Figure 12E, F, G, H; Figure 13). Of note, the use of 4 x 10⁶ cells (Figure 13) as well as the application of an incremental static strain profile (ramp strain) was found to be superior to constant static strain, as it further improved the generation of aligned Schwann cell constructs (Figure 12G, H; Figure 13). The rationale behind this strain regime was to constantly strain the fibrin scaffold by increasing tensile stress by 2% each day, since the effect of constant strain could vanish once fibrin hydrogels adapt to the stretch they are exposed to and become worn out. In contrast, cyclic strain impaired cellular alignment compared with the static protocols (Figure 12C, D), although minor alignment is still achievable when compared to floating controls (Figure 12A, B). Moreover, total Schwann cell length was shorter and the protrusions rather diffusely oriented (Figure 12C, D). These findings are novel and unexpected, since direct mechanical stimulation of Schwann cells embedded in matrices to engineer aligned cellular constructs has never been investigated before, and can therefore significantly contribute to future progressions made in the field of peripheral nerve regeneration. However, some studies investigated the effects of mechanical stimulation applied to cells grown in 2D on gene expression, which will be discussed below.

To analyse the effects of mechanical stimulation on gene expression, qPCR analysis was performed. The most prominent changes were present in expression of the pro-regenerative marker BDNF, which was upregulated approximately 3.6-fold on day 7 when subjected to ramp strain compared to day 0. Concomitantly, the expression of myelin-associated genes Sox10 and MBP was downregulated after 7 days of cultivation (Figure 14). Moreover, gene expression of Schwann cells embedded in fibrin were also compared in the course of the present invention with Schwann cells grown as a monolayer. Results showed very high gene expression changes in almost all genes between 2D and 3D samples, showing that cells cultured in a monolayer do not represent the optimal reference group.

To conclude, the examples of the present invention showed that highly aligned Schwann cell-fibrin constructs can be engineered using mechanical stimulation. First, it was shown that rASCs can be differentiated towards a Schwann cell-like phenotype and that these differentiated cells can be aligned using constant static strain. Subsequently, it was shown that highly pure Schwann cell cultures can be isolated from rat sciatic nerves. Mechanical stimulation experiments using primary Schwann cells revealed that highly aligned Schwann cell constructs can be obtained. However, the examples of the present invention also showed that the degree of alignment strongly depends on the type of mechanical stimulation applied. In particular, especially a ramp strain profile, but also static strain yielded superior alignment of Schwann cells along the axis of strain and induced elongation of cells, while cyclic strain, in contrast, only induced minor alignment of cells. Ultimately, gene expression analysis showed that expression of the pro-regenerative marker BDNF was upregulated after 7 days of cultivation especially in the ramp strain group. However, the exact effects of mechanical stimulation on signal transduction in Schwann cells can be further investigated. Taken together, these highly aligned Schwann cell matrices are promising intraluminal fillers for nerve guidance conduits to accelerate and improve peripheral nerve regeneration.

The present invention therefore discloses the following embodiments:
1. Method for producing a cell construct comprising Schwann cells or Schwann cell-like cells and a biocompatible matrix, wherein Schwann cells or Schwann cell-like cells or precursor cells of Schwann cells or Schwann cell-like cells are contacted with the biocompatible matrix and subjected to cultivation, wherein cultivation is at least partially performed by administering mechanical stimulation on the cells in contact with the biocompatible matrix.
2. Method according to embodiment 1, wherein the biocompatible matrix is selected from synthetic biomaterials, preferably polyglycolic acid (PGA), poly (D,L-lactic-co-glycolic) acid (PLGA), and poly L-lactic acid (PLLA); proteins, preferably extracellular matrix proteins, especially collagen, gelatin, fibrin, keratin, fibronectin, laminin, or silk fibroin; and polysaccharides, preferably heparin sulfate, hyaluronic acid chondroitin sulfate, alginate or chitosan; and mixtures thereof.
3. Method according to embodiment 1 or embodiment 2, wherein the cultivation is performed in a bioreactor.
4. Method according to any one of embodiments 1 to 3, wherein the method comprises the steps of:
   - providing a cell construct of a biocompatible matrix with Schwann cells or Schwann cell-like cells or precursor cells of Schwann cells or Schwann cell-like cells in a bioreactor,
   - cultivation of the cell construct in the bioreactor
   - exposing the cell construct to a mechanical stimulation in the bioreactor during cultivation by fixing the cell construct to a mechanical apparatus which applies strain onto the construct.
5. Method according to any one of embodiments 1 to 4, comprising differentiation of the precursor cells into Schwann cell-like cells, wherein differentiation is at least partially performed during stimulation in the bioreactor.
6. Method according to any one of embodiments 1 to 5, wherein the biocompatible matrix is present in a three-dimensional form, preferably with a support structure resembling the three-dimensional form, especially wherein the three-dimensional form is a three-dimensional cylinder form, a three-dimensional cuboid form, or a three-dimensional ring form.
7. Method according to any one of embodiments 1 to 6, wherein the biocompatible matrix is provided finally as a three-dimensional cell construct in an oblongness shape, preferably in the form of a cylinder or a cuboid.
8. Method according to any one of embodiments 1 to 7, wherein the cell construct is a three-dimensional cell construct with a length, defined as the longest extension of the three-dimensional construct, and a diameter, defined as the longest extension of an axis which lies normal to the length of the construct, wherein the ratio of length to diameter is from 2:1 to 100:1, preferably from 4:1 to 40:1, especially from 5:1 to 20:1.
9. Method according to any one of embodiments 1 to 8, wherein cultivation is performed for a duration of 3 to 60 days, preferably for a duration of 7 to 42 days, especially for a duration of 14 to 28 days.
10. Method according to any one of embodiments 1 to 9, wherein the mechanical stimulation on the cells in contact with the biocompatible matrix during cultivation is performed for a duration of 1 to 60 days, preferably for a duration of 3 to 42 days, especially for a duration of 5 to 14 days; and/or wherein the mechanical stimulation on the cells in contact with the biocompatible matrix during cultivation is performed at least for the time of a single mechanical deformation step to 24 h, preferably from 30 min to 12 h, especially from 1 to 6 h, at least once a day for a duration of 1 to 60 days, preferably for a duration of 3 to 42 days, especially for a duration of 5 to 42 days.
11. Method according to any one of embodiments 1 to 10, wherein the mechanical stimulation on the cells is performed by a static strain treatment or a ramp strain treatment, preferably by a ramp strain treatment.
12. Method according to any one of embodiments 1 to 11, wherein the mechanical stimulation is performed as a strain treatment of 1 to 50 %, preferably of 2 to 30 %, especially of 3 to 20 %, or as a treatment corresponding to a strain treatment of 1 to 50 %, preferably of 2 to 30 %, especially of 3 to 20 %.
13. Method according to any one of embodiments 1 to 12, wherein the cell construct is at least partially exposed to electrical stimulation during cultivation, preferably by applying 10 to 200 mV/mm, more preferably 25 to 150 mV/mm, especially 50 to 125 mV/mm, for 1 to 24 h, more preferably from 2 to 12 h, especially from 3 to 8 h.
14. Method according to any one of embodiments 1 to 13, wherein cultivation and mechanical stimulation are performed until bands of Büngner-like structures have formed.
15. Method according to any one of embodiments 1 to 14, wherein the cell construct is subjected to washing and/or formulation steps for adapting the cell construct to surgical use, preferably by removing the cell construct from a bioreactor after cultivation and stimulation and placing the cell construct in a pharmaceutically acceptable medium.
16. Method according to any one of embodiments 1 to 15, wherein the cell construct is transferred to a transport medium after cultivation and stimulation subjected.
17. Method according to any one of embodiments 1 to 16, wherein the cell construct is brought into a storage-stable form after cultivation and stimulation, preferably by freezing or lyophilizing the cell construct.
18. Method according to any one of embodiments 1 to 17, wherein the cell construct is cultivated in a serum free medium or transferred into a serum free medium after cultivation and stimulation.
19. Method according to any one of embodiments 1 to 18, wherein cultivation and stimulation are performed under aseptic conditions.
20. Method according to any one of embodiments 1 to 19, wherein 250,000 to 15,000,000 cells, preferably from 500,000 to 10,000,000 cells, especially from 1,000,000 to 5,000,000 cells are contacted with the biocompatible matrix and subjected to cultivation.
21. Cell construct obtainable by a method according to any one of embodiments 1 to 20.
22. Cell construct comprising Schwann cells or Schwann cell-like cells and a biocompatible matrix, wherein the cell construct has a bands of Büngner structure.
23. Cell construct according to embodiment 21 or 22, wherein the construct is a nerve guidance graft, especially alone or in combination with a nerve guidance conduit, preferably comprising a pharmaceutically acceptable carrier or being manufactured for neurosurgical use.
24. Cell construct according to any one of embodiments 21 to 23, for use in a therapeutic treatment, preferably for use in neurosurgery, especially for use in the treatment of nerve injuries.
25. Cell construct according to any one of embodiments 21 to 24 for use in neurapraxia, axonotmesis, and neurotmesis, preferably in neurotmesis.
26. Cell construct according to any one of embodiments 21 to 24 for use in the treatment of Degree I to V injuries, preferably of Degree III to V injuries.

## Claims

1. Method for producing a cell construct comprising Schwann cells or Schwann cell-like cells and a biocompatible matrix, wherein Schwann cells or Schwann cell-like cells or precursor cells of Schwann cells or Schwann cell-like cells are contacted with the biocompatible matrix and subjected to cultivation, wherein cultivation is at least partially performed by administering mechanical stimulation on the cells in contact with the biocompatible matrix.

2. Method according to claim 1, wherein the biocompatible matrix is selected from synthetic biomaterials, preferably polyglycolic acid (PGA), poly (D,L-lactic-co-glycolic) acid (PLGA), and poly L-lactic acid (PLLA); proteins, preferably extracellular matrix proteins, especially collagen, gelatin, fibrin, keratin, fibronectin, laminin, or silk fibroin; and polysaccharides, preferably heparin sulfate, hyaluronic acid chondroitin sulfate, alginate or chitosan; and mixtures thereof.

3. Method according to claim 1 or claim 2, wherein the cultivation is performed in a bioreactor.

4. Method according to any one of claims 1 to 3, wherein the biocompatible matrix is present in a three-dimensional form, preferably with a support structure resembling the three-dimensional form, especially wherein the three-dimensional form is a three-dimensional cylinder form, a three-dimensional cuboid form, or a three-dimensional ring form.

5. Method according to any one of claims 1 to 4, wherein cultivation is performed for a duration of 3 to 60 days, preferably for a duration of 7 to 42 days, especially for a duration of 14 to 28 days.

6. Method according to any one of claims 1 to 5, wherein the mechanical stimulation on the cells in contact with the biocompatible matrix during cultivation is performed for a duration of 1 to 60 days, preferably for a duration of 3 to 42 days, especially for a duration of 5 to 14 days.

7. Method according to any one of claims 1 to 6, wherein the mechanical stimulation on the cells is performed by a static strain treatment or a ramp strain treatment, preferably by a ramp strain treatment.

8. Method according to any one of claims 1 to 7, wherein cultivation and mechanical stimulation is performed until bands of Büngner-like structures have formed.

9. Method according to any one of claims 1 to 8, wherein the cell construct has a ratio of length to diameter of from 2:1 to 100:1, preferably from 4:1 to 40:1, especially from 5:1 to 20:1.

10. Method according to any one of claims 1 to 9, wherein the construct is at least partially exposed to electrical stimulation.

11. Cell construct obtainable by a method according to any one of claims 1 to 10.

12. Cell construct comprising Schwann cells or Schwann cell-like cells and a biocompatible matrix, wherein the cell construct has a bands of Büngner structure.

13. Cell construct according to claim 11 or 12, wherein the construct is a nerve guidance graft, especially alone or in combination with a nerve guidance conduit, preferably comprising a pharmaceutically acceptable carrier or being manufactured for neurosurgical use.

14. Cell construct according to any one of claims 11 to 13, for use in a therapeutic treatment, preferably for use in neurosurgery, especially for use in the treatment of nerve injuries.

15. Cell construct according to any one of claims 11 to 14 for use in the treatment of Degree I to V injuries, preferably of Degree III to V injuries.


<110>  FACHHOCHSCHULE TECHNIKUM WIEN

<120>  Cell construct comprising Schwann cells or Schwann cell-like
       cells and a biocompatible matrix

<130>  R 76194

<160>  8

<170>  PatentIn version 3.5

<210>  1
<211>  24
<212>  DNA
<213>  Artificial Sequence

<220>
<223>  prime sequence

<400>  1
tctacgagac caagtgtaat ccca                                              24


<210>  2
<211>  20
<212>  DNA
<213>  Artificial Sequence

<220>
<223>  primer sequence

<400>  2
cttatgaacc gccagccaat                                                   20


<210>  3
<211>  20
<212>  DNA
<213>  Artificial Sequence

<220>
<223>  primer sequence

<400>  3
tgccaaagcc caggtgaaga                                                   20


<210>  4
<211>  23
<212>  DNA
<213>  Artificial Sequence

<220>
<223>  primer sequence

<400>  4
agactgaggg aggtgtaggc gat                                               23


<210>  5
<211>  21

<212>  DNA
<213>  Artificial Sequence

<220>
<223>  primer sequence

<400>  5
gaagtcgcag aggacccaag a                                                21


<210>  6
<211>  20
<212>  DNA
<213>  Artificial Sequence

<220>
<223>  primer sequence

<400>  6
ctgcctccgt agccaaatcc                                                  20


<210>  7
<211>  20
<212>  DNA
<213>  Artificial Sequence

<220>
<223>  primer sequence

<400>  7
ccgtatcgga cgcctggtta                                                  20


<210>  8
<211>  24
<212>  DNA
<213>  Artificial Sequence

<220>
<223>  primer sequence

<400>  8
ccgtgggtag agtcatactg gaac                                             24